# EUROPEAN PATENT SPECIFICATION

(11) **EP 4 513 431 B1**
(45) Date of publication and mention of the grant of the patent: **07.10.2026**
(21) Application number: 24221826.1
(22) Date of filing: 15.10.2020
(51) Int. Cl.: G01N 1/30, G01N 33/483, G01N 33/50, G06T 7/00

(54) **CLINICAL- AND INDUSTRIAL-SCALE INTACT-TISSUE SEQUENCING**
SEQUENZIERUNG VON INTAKTEM GEWEBE IN KLINISCHEM UND INDUSTRIELLEM MASSSTAB
SÉQUENÇAGE DE TISSU INTACT À L'ÉCHELLE CLINIQUE ET INDUSTRIELLE

(30) Priority: 18.10.2019 US 201962923026 P
(43) Date of publication of application: 26.02.2025
(62) Document number(s) of the earlier application(s) in accordance with Art. 76 EPC: 20875765.8 / 4 045 887
(73) Proprietor: The Board of Trustees of the Leland Stanford Junior University, Stanford, CA 94305-2038 (US)
(72) Inventor: RICHMAN, Ethan B., Woodside, California 94062 (US); ALLEN, William E., Albany, California 94706 (US); DEISSEROTH, Karl A., Stanford, California 94305 (US)
(74) Representative: Bassil, Nicholas Charles

(56) References cited:
- WO-A1-2018/033528
- XIAO WANG ET AL: "Three-dimensional intact-tissue sequencing of single-cell transcriptional states", SCIENCE, vol. 361, no. 6400, 27 July 2018 (2018-07-27), US, pages eaat5691, XP055585756, ISSN: 0036-8075, DOI: 10.1126/science.aat5691
- JE HYUK LEE ET AL: "Fluorescent in situ sequencing (FISSEQ) of RNA for gene expression profiling in intact cells and tissues", NATURE PROTOCOLS, vol. 10, no. 3, 12 February 2015 (2015-02-12), GB, pages 442 - 458, XP055272042, ISSN: 1754-2189, DOI: 10.1038/nprot.2014.191

## Description

### STATEMENT REGARDING FEDERALLY SPONSORED RESEARCH OR DEVELOPMENT

This invention was made with Government support under contract W911NF-14-2-0013 awarded by the Defense Advanced Research Projects Agency and under contracts DA042012 and MH075957 awarded by the National Institutes of Health. The Government has certain rights in the invention.

### BACKGROUND OF THE INVENTION

Biological samples contain complex and heterogenous genetic information spanning the length scales of individual cells and whole tissues. Spatial patterns of nucleic acids within a cell may reveal properties and abnormalities of cellular function; cumulative distributions of RNA expression may define a cell type or function; and systematic variation in the locations of cell types within a tissue may define tissue function. The combination of anatomical connectivity information encoded in nucleic acids and tissue-wide cell type distributions may span many sections of tissue. Techniques for *in situ* nucleic acid sequencing must therefore be able to bridge resolutions as small as individual molecules and as large as entire brains. Efficiently collecting and recording this information across orders-of-magnitude differences in lengths requires novel inventions to enhance the robustness, rapidity, automated-, and high throughput-nature of *in situ* sequencing techniques. International Patent Application Publication No. WO 2018033528 A1 describes double-stranded circle probes for detection of a target nucleic acid molecule by an RCA reaction in the presence of the target nucleic acid molecule. Wang et al. (Science (2018) 361(6400):eaat5691) describes devices, methods, and systems for in situ sequencing of target nucleic acids in intact tissue using a method referred to as STARmap (spatially-resolved transcript amplicon readout mapping), which combines hydrogel-tissue chemistry and targeted signal amplification with in situ sequencing. Lee et al. (Na Protoc (2015), 10(3): 442-458) describes fluorescent in situ sequencing (FISSEQ) of RNA for gene expression profiling in intact cells and tissues.

### SUMMARY OF THE INVENTION

The aspects of the invention for which protection is sought are set out in the appended set of claims. Devices, methods, and systems for *in situ* gene sequencing of a target nucleic acid in a cell in an intact tissue are provided. Methods of screening a candidate agent to determine whether the candidate agent modulates gene expression of a nucleic acid in a cell in an intact tissue are also provided herein.

In one aspect, a method for *in situ* gene sequencing of a target nucleic acid in a cell in an intact tissue is provided, the method comprising: (a) contacting a fixed and permeabilized intact tissue sample with at least a pair of oligonucleotides under conditions to allow for specific hybridization, wherein the pair of oligonucleotides comprises a first oligonucleotide and a second oligonucleotide; wherein each of the first oligonucleotide and the second oligonucleotide comprises a first complementarity region, a second complementarity region, and a third complementarity region; wherein the second oligonucleotide further comprises a barcode sequence; wherein the first complementarity region of the first oligonucleotide is complementary to a first portion of the target nucleic acid, wherein the second complementarity region of the first oligonucleotide is complementary to the first complementarity region of the second oligonucleotide, wherein the third complementarity region of the first oligonucleotide is complementary to the third complementarity region of the second oligonucleotide, wherein the second complementary region of the second oligonucleotide is complementary to a second portion of the target nucleic acid, and wherein the first complementarity region of the first oligonucleotide is adjacent to the second complementarity region of the second oligonucleotide; (b) adding ligase to ligate the second oligonucleotide 5' and 3' ends to generate a closed nucleic acid circle; (c) preincubating the tissue sample with a DNA polymerase for a sufficient time to allow uniform diffusion of the DNA polymerase throughout the tissue sample before performing rolling circle amplification; (d) performing rolling circle amplification by contacting the tissue sample with deoxynucleotide triphosphates such that one or more amplicons are generated, wherein the ligated second oligonucleotide serves as a template and the first oligonucleotide serves as a primer for the DNA polymerase; (e) embedding the tissue sample in a hydrogel before or after any one of steps (a)-(d); (f) cross-linking the one or more amplicons to the hydrogel; (g) clearing the hydrogel to enhance hydrogel transparency; (h) contacting the one or more hydrogel-embedded amplicons having the barcode sequence with a read primer and a fluorescently labeled probe, wherein the probe comprises an oligonucleotide comprising a first thiol group covalently linked to a fluorophore comprising a second thiol group by a disulfide bond between the first thiol group and the second thiol group; (i) ligating the read primer and the fluorescently labeled probe, wherein the ligation only occurs when both the read primer and the fluorescently labeled probe are complementary to adjacent sequences of the same amplicon; (j) contacting the hydrogel with an anti-fade buffer comprising an antioxidant; (k) imaging the one or more hydrogel-embedded amplicons to determine positioning of the target nucleic acid in the cell in the intact tissue that is undergoing *in situ* gene sequencing, wherein imaging is performed in presence of the anti-fade buffer; (l) contacting the hydrogel with a reducing agent resulting in reduction of the disulfide bond and cleavage of the fluorophore from the probe; (m) removing the fluorophore from the hydrogel; and (n) reiterating steps (h)-(m).

In certain embodiments, the method further comprises sectioning the intact tissue and placing a section of the intact tissue flat onto a surface. For example, a metal ring can be pressed down onto the section and used to transfer the section flat onto the surface. In some embodiments, cryosectioning is performed on the tissue.

In another aspect, a fluidic system is provided for automation of the methods described herein, allowing for continual operation. In some embodiments, the system includes a fluidics device, and a processor configured to perform the methods described herein.

In certain embodiments, a fluidic system is provided comprising: a) a device comprising an unpressurized sample chamber; b) a lid, wherein the lid covers the top of the sample chamber; c) an interface tube, wherein a first end of the interface tube is held by the lid, and a second end of the interface tube is positioned above a tissue sample in the sample chamber sufficiently close to a bottom edge of the sample chamber such that liquid within the sample chamber remains in contact with the bottom of the interface tube as long as liquid remains in the sample chamber; d) a fluidic line coupled to the first end of the interface tube such that fluid flows through the fluidic line into the interface tube and flows out of the second end of the interface tube onto the tissue sample in the sample chamber; e) a pump; f) an imaging system; and g) a processor unit configured to perform the methods described herein. In some embodiments, the sample chamber is a well of a multiwell plate or a slide chamber. In some embodiments, the interface tube is placed at a polar angle relative to the tissue sample plane. In some embodiments, the fluidic system further comprises a cryostat that maintains the sample chamber at a cryogenic temperature. In some embodiments, the fluidic system further comprises a multiway selector valve interfaced with the pump. In some embodiments, the fluidic system further comprises a reagent tray comprising one or more containers or wells comprising reagents for performing the methods described herein, wherein the reagents are fluidically connected to the multiway selector valve. In some embodiments, the fluidic system further comprises a buffer tray comprising one or more containers or wells comprising buffers for performing the methods described herein, wherein the buffers are fluidically connected to the multiway selector valve. In some embodiments, the reagent tray and/or the buffer tray are disposable. In some embodiments, the fluidic system further comprises an adjustable stage supporting the reagent tray, a chiller, and a position sensor that allows movement of the adjustable stage to allow selection of a reagent from the reagent tray. In some embodiments, the fluidic system further comprises a waste container fluidically connected to the multiway selector valve. In some embodiments, the imaging system comprises a microscope that performs confocal microscopy, spinning disk confocal microscopy, light sheet microscopy, lattice light sheet microscopy, or light field microscopy. In some embodiments, the fluidic system further comprises a container enclosing the fluidic system, wherein the container blocks ambient light. In some embodiments, the container enclosing the fluidic system comprises one or more doors or drawers. In some embodiments, the container enclosing the fluidic system is an acoustic dampening container.

### BRIEF DESCRIPTION OF THE DRAWINGS

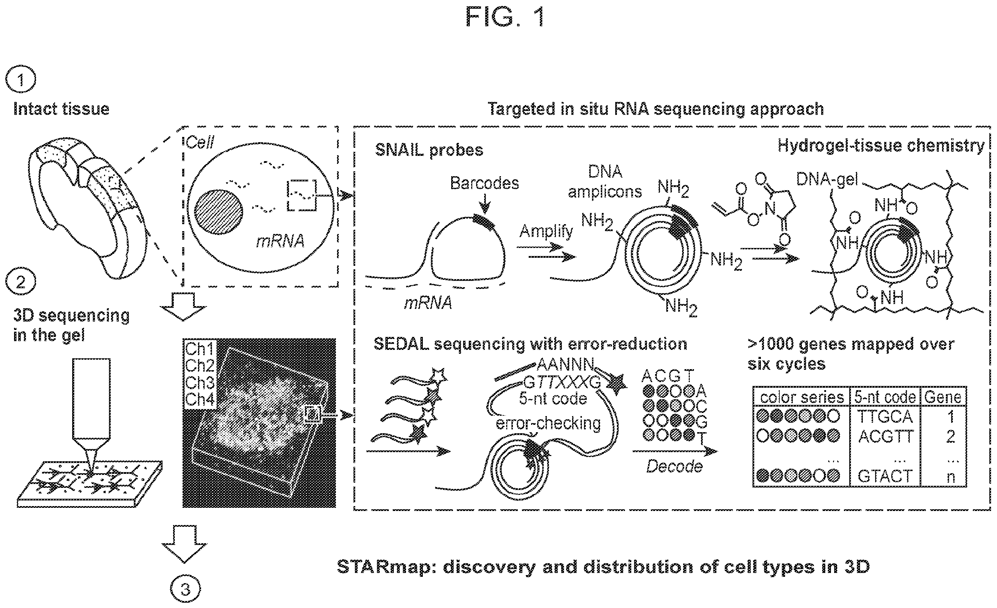

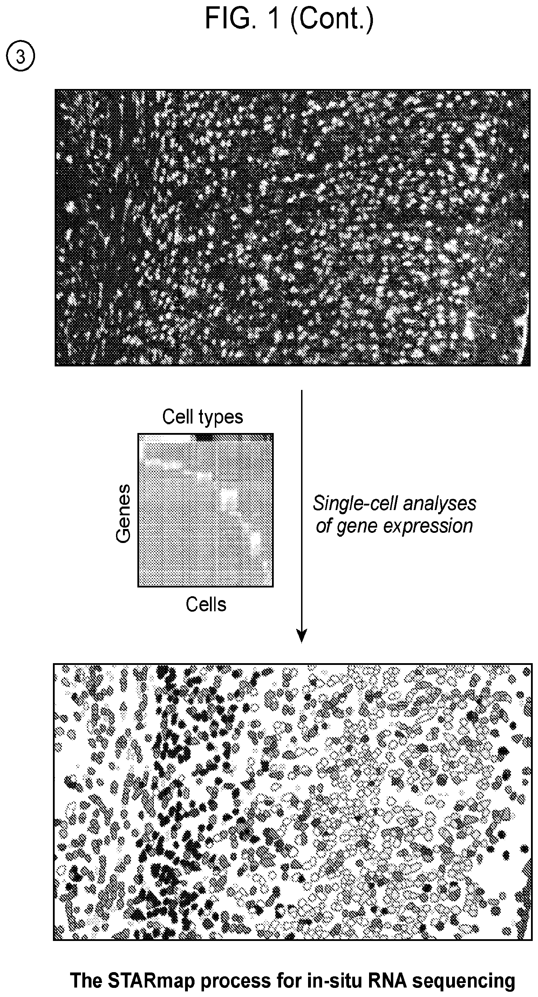

FIG. 1 shows intact tissue sequencing - integrating anatomical and genomic data.

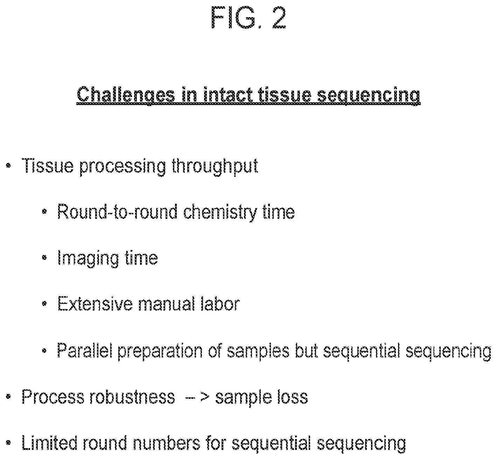

FIG. 2 shows challenges in intact tissue sequencing.

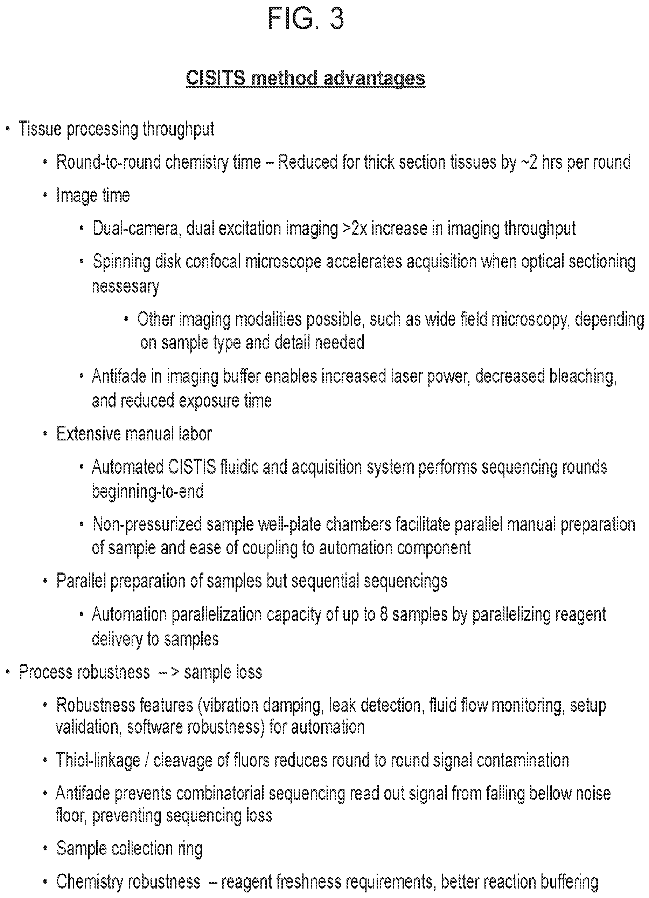

FIG. 3 shows CISITS method advantages.

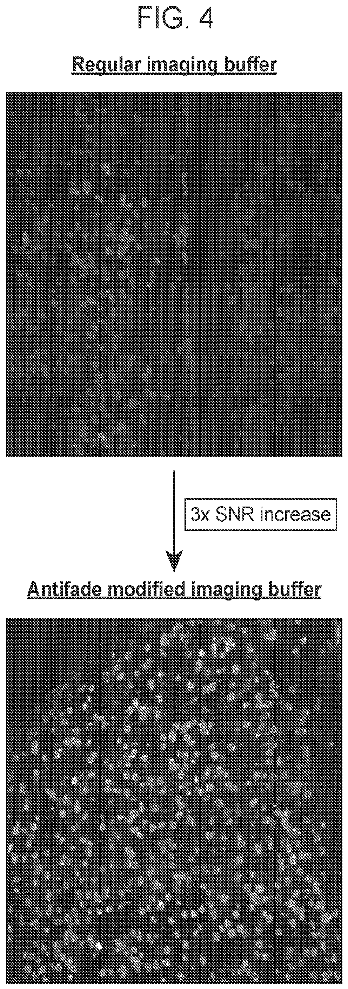

FIG. 4 shows antifade with propyl-gallate or other antioxidant reduces photobleaching.

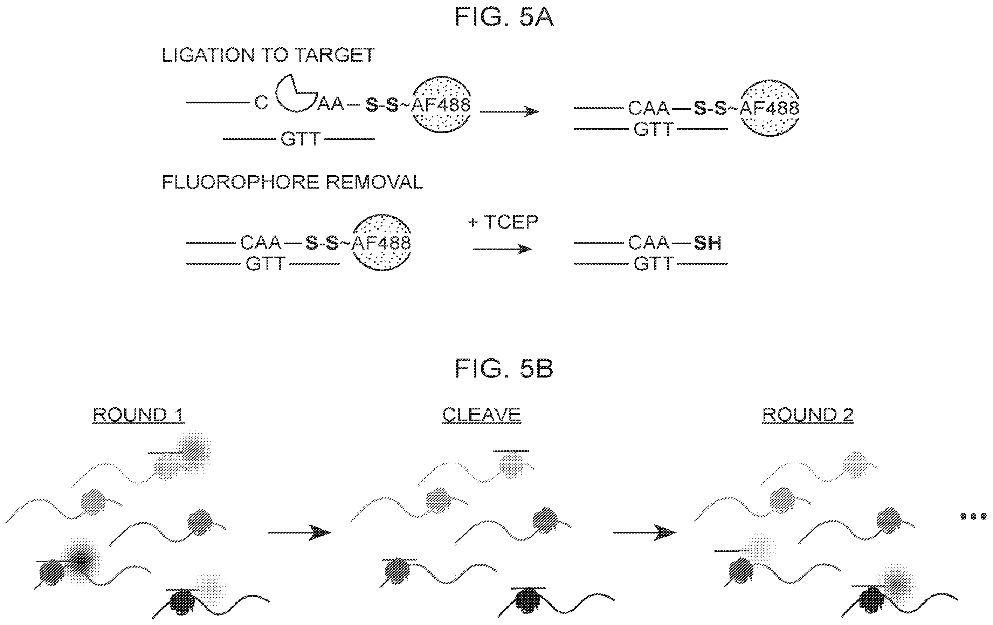

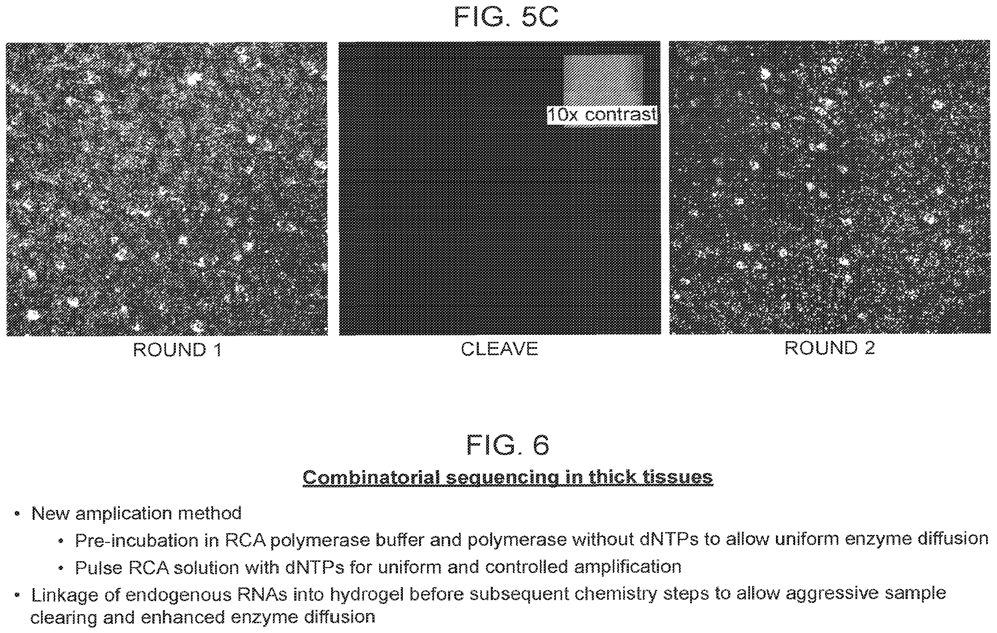

FIG. 5 shows round-to-round robustness with thiol-mediated read-out fluor probes for sequential sequencing.
FIG. 6 shows combinatorial sequencing in thick tissues.
FIG. 7 shows CISITS sequencer diagram.

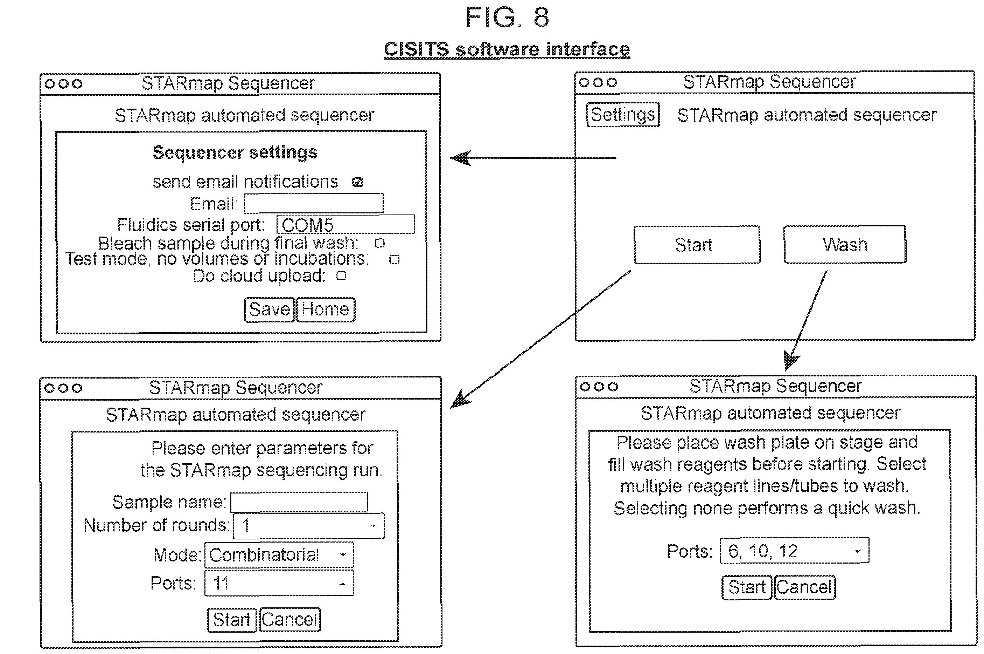

FIG. 8 shows CISITS software interface.

### DETAILED DESCRIPTION OF THE INVENTION

Provided herein are devices, methods, and systems for *in situ* gene sequencing of a target nucleic acid in a cell in an intact tissue. Methods of screening a candidate agent to determine whether the candidate agent modulates gene expression of a nucleic acid in a cell in an intact tissue are also provided herein.

Before the present devices, methods, and systems are described, it is to be understood that this invention is not limited to particular methods or compositions described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within the invention. The upper and lower limits of these smaller ranges may independently be included or excluded in the range, and each range where either, neither or both limits are included in the smaller ranges is also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, some potential and preferred methods and materials are now described.

It must be noted that as used herein and in the appended claims, the singular forms "a", "an", and "the" include plural referents unless the context clearly dictates otherwise. Thus, for example, reference to "a cell" includes a plurality of such cells and reference to "the peptide" includes reference to one or more peptides and equivalents thereof, e.g. oligopeptides or polypeptides known to those skilled in the art, and so forth.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

### Definitions

The term "about", particularly in reference to a given quantity, is meant to encompass deviations of plus or minus five percent.

The terms "peptide", "oligopeptide", "polypeptide", and "protein" are used interchangeably herein to refer to a polymer of amino acid residues. The terms also apply to amino acid polymers in which one or more amino acid residue is an artificial chemical mimetic of a corresponding naturally occurring amino acid, as well as to naturally occurring amino acid polymers and non-naturally occurring amino acid polymers. Both full-length proteins and fragments thereof are encompassed by the definition. The terms also include post-expression modifications of the polypeptide, for example, phosphorylation, glycosylation, acetylation, hydroxylation, oxidation, and the like as well as chemically or biochemically modified or derivatized amino acids and polypeptides having modified peptide backbones. The terms also include fusion proteins, including, but not limited to, fusion proteins with a heterologous amino acid sequence, fusions with heterologous and homologous leader sequences, with or without N-terminal methionine residues; immunologically tagged proteins; and the like. The terms include polypeptides including one or more of a fatty acid moiety, a lipid moiety, a sugar moiety, and a carbohydrate moiety.

As used herein, the term "target nucleic acid" is any polynucleotide nucleic acid molecule (e.g., DNA molecule; RNA molecule, modified nucleic acid, etc.) present in a single cell. In some embodiments, the target nucleic acid is a coding RNA (e.g., mRNA). In some embodiments, the target nucleic acid is a non-coding RNA (e.g., tRNA, rRNA, microRNA (miRNA), mature miRNA, immature miRNA; etc.). In some embodiments, the target nucleic acid is a splice variant of an RNA molecule (e.g., mRNA, pre-mRNA, etc.) in the context of a cell. A suitable target nucleic acid can therefore be an unspliced RNA (e.g., pre-mRNA, mRNA), a partially spliced RNA, or a fully spliced RNA, etc. Target nucleic acids of interest may be variably expressed, i.e. have a differing abundance, within a cell population, wherein the methods of the invention allow profiling and comparison of the expression levels of nucleic acids, including without limitation RNA transcripts, in individual cells. A target nucleic acid can also be a DNA molecule, e.g. a denatured genomic, viral, plasmid, etc. For example, the methods can be used to detect copy number variants, e.g. in a cancer cell population in which a target nucleic acid is present at different abundance in the genome of cells in the population; a virus-infected cells to determine the virus load and kinetics, and the like.

The terms "oligonucleotide," "polynucleotide," and "nucleic acid molecule", used interchangeably herein, refer to polymeric forms of nucleotides of any length, either ribonucleotides or deoxyribonucleotides. Thus, this term includes, but is not limited to, single-, double-, or multi-stranded DNA or RNA, genomic DNA, cDNA, DNA-RNA hybrids, or a polymer including purine and pyrimidine bases or other natural, chemically or biochemically modified, non-natural, or derivatized nucleotide bases. The backbone of the polynucleotide can include sugars and phosphate groups (as may typically be found in RNA or DNA), or modified or substituted sugar or phosphate groups. Alternatively, the backbone of the polynucleotide can include a polymer of synthetic subunits such as phosphoramidites, and/or phosphorothioates, and thus can be an oligodeoxynucleoside phosphoramidate or a mixed phosphoramidate-phosphodiester oligomer. Peyrottes et al. (1996) Nucl. Acids Res. 24:1841-1848; Chaturvedi et al. (1996) Nucl. Acids Res. 24:2318-2323. The polynucleotide may include one or more L-nucleosides. A polynucleotide may include modified nucleotides, such as methylated nucleotides and nucleotide analogs, uracyl, other sugars, and linking groups such as fluororibose and thioate, and nucleotide branches. The sequence of nucleotides may be interrupted by non-nucleotide components. A polynucleotide may be modified to include N3'-P5' (NP) phosphoramidate, morpholino phosphorociamidate (MF), locked nucleic acid (LNA), 2'-O-methoxyethyl (MOE), or 2'-fluoro, arabino-nucleic acid (FANA), which can enhance the resistance of the polynucleotide to nuclease degradation (see, e.g., Faria et al. (2001) Nature Biotechnol. 19:40-44; Toulme (2001) Nature Biotechnol. 19:17-18). A polynucleotide may be further modified after polymerization, such as by conjugation with a labeling component. Other types of modifications included in this definition are caps, substitution of one or more of the naturally occurring nucleotides with an analog, and introduction of means for attaching the polynucleotide to proteins, metal ions, labeling components, other polynucleotides, or a solid support. Immunomodulatory nucleic acid molecules can be provided in various formulations, e.g., in association with liposomes, microencapsulated, etc., as described in more detail herein. A polynucleotide used in amplification is generally single-stranded for maximum efficiency in amplification, but may alternatively be double-stranded. If double-stranded, the polynucleotide can first be treated to separate its strands before being used to prepare extension products. This denaturation step is typically affected by heat, but may alternatively be carried out using alkali, followed by neutralization.

By "isolated" is meant, when referring to a protein, polypeptide, or peptide, that the indicated molecule is separate and discrete from the whole organism with which the molecule is found in nature or is present in the substantial absence of other biological macro molecules of the same type. The term "isolated" with respect to a polynucleotide is a nucleic acid molecule devoid, in whole or part, of sequences normally associated with it in nature; or a sequence, as it exists in nature, but having heterologous sequences in association therewith; or a molecule disassociated from the chromosome.

The terms "subject", "individual" or "patient" are used interchangeably herein and refer to a vertebrate, preferably a mammal. By "vertebrate" is meant any member of the subphylum chordata, including, without limitation, humans and other primates, including non-human primates such as chimpanzees and other apes and monkey species; farm animals such as cattle, sheep, pigs, goats and horses; domestic mammals such as dogs and cats; laboratory animals including rodents such as mice, rats and guinea pigs; birds, including domestic, wild and game birds such as chickens, turkeys and other gallinaceous birds, ducks, geese, and the like. The term does not denote a particular age. Thus, both adult and newborn individuals are intended to be covered.

### Methods

The methods disclosed herein include the use of a modified Spatially-resolved Transcript Amplicon Readout Mapping (STARmap) technique, termed "Clinical and Industrial Scale Intact Tissue Sequencing" (CISITS), which improves both the sequencing chemistry and the automated execution of sequencing. For a description of the original STARmap technique, see, e.g., International Patent Application Publication No. WO2019/199579A1 and Wang et al. (2018) Science 361(6400):eaat5691. Like STARmap, CISITS utilizes an image-based *in situ* nucleic acid (DNA and/or RNA) sequencing technology using a sequencing-by-ligation process, specific signal amplification, hydrogel-tissue chemistry to turn biological tissue into a transparent sequencing chip, and associated data analysis pipelines to spatially-resolve highly-multiplexed gene detection at a subcellular and cellular level. CISITS adds improvements that increase the signal-to-noise ratio (SNR) of labeled molecules, eliminates carryover signal across rounds, and makes possible combinatorial sequencing in thick tissues. Also disclosed herein is a fluidic system for automation of the CISITS methods that allows parallelization across samples. The fluidic system includes a sample chamber that is no longer pressurized and also includes CISITS integration with microscope hardware. Additionally, CISITS software to control the automated fluidic system is provided.

As summarized above, the methods disclosed herein include a method for *in situ* gene sequencing of a target nucleic acid in a cell in an intact tissue. The method typically comprises: (a) contacting a fixed and permeabilized intact tissue sample with at least a pair of oligonucleotides under conditions to allow for specific hybridization, wherein the pair of oligonucleotides comprises a first oligonucleotide and a second oligonucleotide; wherein each of the first oligonucleotide and the second oligonucleotide comprises a first complementarity region, a second complementarity region, and a third complementarity region; wherein the second oligonucleotide further comprises a barcode sequence; wherein the first complementarity region of the first oligonucleotide is complementary to a first portion of the target nucleic acid, wherein the second complementarity region of the first oligonucleotide is complementary to the first complementarity region of the second oligonucleotide, wherein the third complementarity region of the first oligonucleotide is complementary to the third complementarity region of the second oligonucleotide, wherein the second complementary region of the second oligonucleotide is complementary to a second portion of the target nucleic acid, and wherein the first complementarity region of the first oligonucleotide is adjacent to the second complementarity region of the second oligonucleotide; (b) adding ligase to ligate the second oligonucleotide 5' and 3' ends to generate a closed nucleic acid circle; (c) preincubating the tissue sample with a DNA polymerase for a sufficient time to allow uniform diffusion of the DNA polymerase throughout the tissue sample before performing rolling circle amplification; (d) performing rolling circle amplification by contacting the tissue sample with deoxynucleotide triphosphates such that one or more amplicons are generated, wherein the ligated second oligonucleotide serves as a template and the first oligonucleotide serves as a primer for the DNA polymerase; (e) embedding the tissue sample in a hydrogel before or after any one of steps (a)-(d); (f) cross-linking the one or more amplicons to the hydrogel; (g) clearing the hydrogel to enhance hydrogel transparency; (h) contacting the one or more hydrogel-embedded amplicons having the barcode sequence with a read primer and a fluorescently labeled probe, wherein the probe comprises an oligonucleotide comprising a first thiol group covalently linked to a fluorophore comprising a second thiol group by a disulfide bond between the first thiol group and the second thiol group; (i) ligating the read primer and the fluorescently labeled probe, wherein the ligation only occurs when both the read primer and the fluorescently labeled probe are complementary to adjacent sequences of the same amplicon; (j) contacting the hydrogel with an anti-fade buffer comprising an antioxidant; (k) imaging the one or more hydrogel-embedded amplicons to determine positioning of the target nucleic acid in the cell in the intact tissue that is undergoing *in situ* gene sequencing, wherein imaging is performed in presence of the anti-fade buffer; (l) contacting the hydrogel with a reducing agent resulting in reduction of the disulfide bond and cleavage of the fluorophore from the probe; (m) removing the fluorophore from the hydrogel; and (n) reiterating steps (h)-(m).

The methods disclosed herein also provide for a method of screening a candidate agent to determine whether the candidate agent modulates gene expression of a nucleic acid in a cell in an intact tissue, the method including: (a) contacting a fixed and permeabilized intact tissue with at least a pair of oligonucleotide primers under conditions to allow for specific hybridization, wherein the pair of primers include a first oligonucleotide and a second oligonucleotide; wherein each of the first oligonucleotide and the second oligonucleotide includes a first complementarity region, a second complementarity region, and a third complementarity region; wherein the second oligonucleotide further includes a barcode sequence; wherein the first complementarity region of the first oligonucleotide is complementary to a first portion of the target nucleic acid, wherein the second complementarity region of the first oligonucleotide is complementary to the first complementarity region of the second oligonucleotide, wherein the third complementarity region of the first oligonucleotide is complementary to the third complementarity region of the second oligonucleotide, wherein the second complementary region of the second oligonucleotide is complementary to a second portion of the target nucleic acid, and wherein the first complementarity region of the first oligonucleotide is adjacent to the second complementarity region of the second oligonucleotide; (b) adding ligase to ligate the second oligonucleotide and generate a closed nucleic acid circle; (c) performing rolling circle amplification in the presence of a nucleic acid molecule, wherein the performing includes using the second oligonucleotide as a template and the first oligonucleotide as a primer for a polymerase to form one or more amplicons; (d) embedding the one or more amplicons in the presence of hydrogel subunits to form one or more hydrogel-embedded amplicons; (e) contacting the one or more hydrogel-embedded amplicons having the barcode sequence with a pair of primers under conditions to allow for ligation, wherein the pair of primers include a third oligonucleotide and a fourth oligonucleotide, wherein the ligation only occurs when both the third oligonucleotide and the fourth oligonucleotide ligate to the same amplicon; (f) reiterating step (e); (g) imaging the one or more hydrogel-embedded amplicons to determine *in situ* gene sequencing of the target nucleic acid in the cell in the intact tissue; and (h) detecting the level of gene expression of the target nucleic acid, wherein an alteration in the level of expression of the target nucleic acid in the presence of the at least one candidate agent relative to the level of expression of the target nucleic acid in the absence of the at least one candidate agent indicates that the at least one candidate agent modulates gene expression of the nucleic acid in the cell in the intact tissue.

In certain aspects, the methods disclosed herein provide for a faster processing time, higher multiplexity (up to 1000 genes), higher efficiency, higher sensitivity, lower error rate, and more spatially resolved cell types, as compared to existing gene expression analysis tools. In such aspects, the improved hydrogel-tissue chemistry method transforms biological tissue into nucleic acids imprinted with hydrogel compatible with *in situ* sequencing, an improved sequencing-by-ligation process (SEDAL) for *in situ* sequencing with error reduction. In some other aspects, the methods disclosed herein include spatially sequencing (e.g. reagents, chips or services) for biomedical research and clinical diagnostics (e.g. cancer, bacterial infection, viral infection, etc.) with single-cell and/or single-molecule sensitivity.

### Specific Amplification of Nucleic Acids via Intramolecular Ligation (SNAIL)

In some embodiments, one component of CISITS includes an efficient approach for generating cDNA libraries from cellular RNAs *in situ,* which may be referred to as SNAIL, for Specific Amplification of Nucleic Acids via Intramolecular Ligation. In certain embodiments, the subject methods include contacting a fixed and permeabilized intact tissue with at least a pair of oligonucleotide primers under conditions to allow for specific hybridization, wherein the pair of primers includes a first oligonucleotide and a second oligonucleotide.

More generally, the nucleic acid present in a cell of interest in a tissue serves as a scaffold for an assembly of a complex that includes a pair of primers, referred to herein as a first oligonucleotide and a second oligonucleotide. In some embodiments, the contacting the fixed and permeabilized intact tissue includes hybridizing the pair of primers to the same target nucleic acid. In some embodiments, the target nucleic acid is RNA. In such embodiments, the target nucleic acid may be mRNA. In other embodiments, the target nucleic acid is DNA.

As used herein, the terms "hybridize" and "hybridization" refer to the formation of complexes between nucleotide sequences which are sufficiently complementary to form complexes via Watson-Crick base pairing. Where a primer "hybridizes" with target (template), such complexes (or hybrids) are sufficiently stable to serve the priming function required by, e.g., the DNA polymerase to initiate DNA synthesis. It will be appreciated that the hybridizing sequences need not have perfect complementarity to provide stable hybrids. In many situations, stable hybrids will form where fewer than about 10% of the bases are mismatches, ignoring loops of four or more nucleotides. Accordingly, as used herein the term "complementary" refers to an oligonucleotide that forms a stable duplex with its "complement" under assay conditions, generally where there is about 90% or greater homology.

### SNAIL Oligonucleotide Primers

In the method of the invention, the SNAIL oligonucleotide primers include at least a first oligonucleotide and a second oligonucleotide; wherein each of the first oligonucleotide and the second oligonucleotide includes a first complementarity region, a second complementarity region, and a third complementarity region; wherein the second oligonucleotide further includes a barcode sequence; wherein the first complementarity region of the first oligonucleotide is complementary to a first portion of the target nucleic acid, wherein the second complementarity region of the first oligonucleotide is complementary to the first complementarity region of the second oligonucleotide, wherein the third complementarity region of the first oligonucleotide is complementary to the third complementarity region of the second oligonucleotide, wherein the second complementary region of the second oligonucleotide is complementary to a second portion of the target nucleic acid, and wherein the first complementarity region of the first oligonucleotide is adjacent to the second complementarity region of the second oligonucleotide. In an alternative embodiment, the second oligonucleotide is a closed circular molecule, and a ligation step is omitted.

The present disclosure provides methods where the contacting a fixed and permeabilized tissue includes hybridizing a plurality of oligonucleotide primers having specificity for different target nucleic acids. In some embodiments, the methods include a plurality of first oligonucleotides, including, but not limited to, 5 or more first oligonucleotides, e.g., 8 or more, 10 or more, 12 or more, 15 or more, 18 or more, 20 or more, 25 or more, 30 or more, 35 or more that hybridize to target nucleotide sequences. In some embodiments, a method of the present disclosure includes a plurality of first oligonucleotides, including, but not limited to, 15 or more first oligonucleotides, e.g., 20 or more, 30 or more, 40 or more, 50 or more, 60 or more, 70 or more, and up to 80 different first oligonucleotides that hybridize to 15 or more, e.g., 20 or more, 30 or more, 40 or more, 50 or more, 60 or more, 70 or more, and up to 80 different target nucleotide sequences. In some embodiments, the methods include a plurality of second oligonucleotides, including, but not limited to, 5 or more second oligonucleotides, e.g., 8 or more, 10 or more, 12 or more, 15 or more, 18 or more, 20 or more, 25 or more, 30 or more, 35 or more. In some embodiments, a method of the present disclosure includes a plurality of second oligonucleotides including, but not limited to, 15 or more second oligonucleotides, e.g., 20 or more, 30 or more, 40 or more, 50 or more, 60 or more, 70 or more, and up to 80 different first oligonucleotides that hybridize to 15 or more, e.g., 20 or more, 30 or more, 40 or more, 50 or more, 60 or more, 70 or more, and up to 80 different target nucleotide sequences. A plurality of oligonucleotide pairs can be used in a reaction, where one or more pairs specifically bind to each target nucleic acid. For example, two primer pairs can be used for one target nucleic acid in order to improve sensitivity and reduce variability. It is also of interest to detect a plurality of different target nucleic acids in a cell, e.g. detecting up to 2, up to 3, up to 4, up to 5, up to 6, up to 7, up to 8, up to 9, up to 10, up to 12, up to 15, up to 18, up to 20, up to 25, up to 30, up to 40 or more distinct target nucleic acids. The primers are typically denatured prior to use, typically by heating to a temperature of at least about 50°C, at least about 60°C, at least about 70°C, at least about 80°C, and up to about 99°C, up to about 95°C, up to about 90°C.

In some embodiments, the primers are denatured by heating before contacting the sample. In certain aspects, the melting temperature (Tₘ) of oligonucleotides is selected to minimize ligation in solution. The "melting temperature" or "Tm" of a nucleic acid is defined as the temperature at which half of the helical structure of the nucleic acid is lost due to heating or other dissociation of the hydrogen bonding between base pairs, for example, by acid or alkali treatment, or the like. The Tₘ of a nucleic acid molecule depends on its length and on its base composition. Nucleic acid molecules rich in GC base pairs have a higher Tₘ than those having an abundance of AT base pairs. Separated complementary strands of nucleic acid spontaneously reassociate or anneal to form duplex nucleic acid when the temperature is lowered below the Tₘ. The highest rate of nucleic acid hybridization occurs approximately 25 degrees C below the Tₘ. The Tₘ may be estimated using the following relationship: Tₘ = 69.3 + 0.41(GC)% (Marmur et al. (1962) J. Mol. Biol. 5:109-118).

In certain embodiments, the plurality of second oligonucleotides includes a padlock probe. In some embodiments, the probe includes a detectable label that can be measured and quantitated. The terms "label" and "detectable label" refer to a molecule capable of detection, including, but not limited to, radioactive isotopes, fluorescers, chemiluminescers, enzymes, enzyme substrates, enzyme cofactors, enzyme inhibitors, chromophores, dyes, metal ions, metal sols, ligands (e.g., biotin or haptens) and the like. The term "fluorescer" refers to a substance or a portion thereof that is capable of exhibiting fluorescence in the detectable range. Particular examples of labels that may be used with the invention include, but are not limited to phycoerythrin, Alexa dyes, fluorescein, YPet, CyPet, Cascade blue, allophycocyanin, Cy3, Cy5, Cy7, rhodamine, dansyl, umbelliferone, Texas red, luminol, acradimum esters, biotin, green fluorescent protein (GFP), enhanced green fluorescent protein (EGFP), yellow fluorescent protein (YFP), enhanced yellow fluorescent protein (EYFP), blue fluorescent protein (BFP), red fluorescent protein (RFP), firefly luciferase, Renilla luciferase, NADPH, beta-galactosidase, horseradish peroxidase, glucose oxidase, alkaline phosphatase, chloramphenical acetyl transferase, and urease.

In some embodiments, the one or more first oligonucleotides and second oligonucleotides bind to a different region of the target nucleic acid, or target site. In a pair, each target site is different, and the target sites are adjacent sites on the target nucleic acid, e.g. usually not more than 15 nucleotides distant, e.g. not more than 10, 8, 6, 4, or 2 nucleotides distant from the other site, and may be contiguous sites. Target sites are typically present on the same strand of the target nucleic acid in the same orientation. Target sites are also selected to provide a unique binding site, relative to other nucleic acids present in the cell. Each target site is generally from about 19 to about 25 nucleotides in length, e.g. from about 19 to 23 nucleotides, from about 19 to 21 nucleotides, or from about 19 to 20 nucleotides. The pair of first and second oligonucleotides are selected such that each oligonucleotide in the pair has a similar melting temperature for binding to its cognate target site, e.g. the Tₘ may be from about 50°C, from about 52°C, from about 55°C, from about 58°, from about 62°C, from about 65°C, from about 70°C, or from about 72°C. The GC content of the target site is generally selected to be no more than about 20%, no more than about 30%, no more than about 40%, no more than about 50%, no more than about 60%, no more than about 70%,

In some embodiments, the first oligonucleotide includes a first, second, and third complementarity region. The target site of the first oligonucleotide may refer to the first complementarity region. As summarized above, the first complementarity region of the first oligonucleotide may have a length of 19-25 nucleotides. In certain aspects, the second complementarity region of the first oligonucleotide has a length of 3-10 nucleotides, including, e.g., 4-8 nucleotides or 4-7 nucleotides. In some aspects, the second complementarity region of the first oligonucleotide has a length of 6 nucleotides. In some embodiments, the third complementarity region of the first oligonucleotide likewise has a length of 6 nucleotides. In such embodiments, the third complementarity region of the first oligonucleotide has a length of 3-10 nucleotides, including, e.g., 4-8 nucleotides or 4-7 nucleotides.

In some embodiments, second first oligonucleotide includes a first, second, and third complementarity region. The target site of the second oligonucleotide may refer to the second complementarity region. As summarized above, the second complementarity region of the second oligonucleotide may have a length of 19-25 nucleotides. In certain aspects, the first complementarity region of the first oligonucleotide has a length of 3-10 nucleotides, including, e.g., 4-8 nucleotides or 4-7 nucleotides. In some aspects, the first complementarity region of the first oligonucleotide has a length of 6 nucleotides. In some aspects, the first complementarity region of the second oligonucleotide includes the 5' end of the second oligonucleotide.

In some embodiments, the third complementarity region of the second oligonucleotide likewise has a length of 6 nucleotides. In such embodiments, the third complementarity region of the second oligonucleotide has a length of 3-10 nucleotides, including, e.g., 4-8 nucleotides or 4-7 nucleotides. In further embodiments, the third complementarity region of the second oligonucleotide includes the 3' end of the second oligonucleotide. In some embodiments, the first complementarity region of the second oligonucleotide is adjacent to the third complementarity region of the second oligonucleotide.

In some aspects, the second oligonucleotide includes a barcode sequence, wherein the barcode sequence of the second oligonucleotide provides barcoding information for identification of the target nucleic acid. The term "barcode" refers to a nucleic acid sequence that is used to identify a single cell or a subpopulation of cells. Barcode sequences can be linked to a target nucleic acid of interest during amplification and used to trace back the amplicon to the cell from which the target nucleic acid originated. A barcode sequence can be added to a target nucleic acid of interest during amplification by carrying out amplification with an oligonucleotide that contains a region including the barcode sequence and a region that is complementary to the target nucleic acid such that the barcode sequence is incorporated into the final amplified target nucleic acid product (i.e., amplicon).

### Tissue

As described herein, the methods disclosed include *in situ* sequencing technology of an intact tissue by at least contacting a fixed and permeabilized intact tissue with at least a pair of oligonucleotide primers under conditions to allow for specific hybridization. Tissue specimens suitable for use with the methods described herein generally include any type of tissue specimens collected from living or dead subjects, such as, e.g., biopsy specimens and autopsy specimens, of which include, but are not limited to, epithelium, muscle, connective, and nervous tissue. Tissue specimens may be collected and processed using the methods described herein and subjected to microscopic analysis immediately following processing, or may be preserved and subjected to microscopic analysis at a future time, e.g., after storage for an extended period of time. In some embodiments, the methods described herein may be used to preserve tissue specimens in a stable, accessible and fully intact form for future analysis. In some embodiments, the methods described herein may be used to analyze a previously-preserved or stored tissue specimen. In some embodiments, the intact tissue includes brain tissue such as visual cortex slices. In some embodiments, the intact tissue is a thin slice with a thickness of 5-20 µm, including, but not limited to, e.g., 5-18 µm, 5-15 µm, or 5-10 µm. In other embodiments, the intact tissue is a thick slice with a thickness of 50-200 µm, including, but not limited to, e.g., 50-150 µm, 50-100 µm, or 50-80 µm.

Aspects of the invention include fixing intact tissue. The term "fixing" or "fixation" as used herein is the process of preserving biological material (e.g., tissues, cells, organelles, molecules, etc.) from decay and/or degradation. Fixation may be accomplished using any convenient protocol. Fixation can include contacting the sample with a fixation reagent (i.e., a reagent that contains at least one fixative). Samples can be contacted by a fixation reagent for a wide range of times, which can depend on the temperature, the nature of the sample, and on the fixative(s). For example, a sample can be contacted by a fixation reagent for 24 or less hours, 18 or less hours, 12 or less hours, 8 or less hours, 6 or less hours, 4 or less hours, 2 or less hours, 60 or less minutes, 45 or less minutes, 30 or less minutes, 25 or less minutes, 20 or less minutes, 15 or less minutes, 10 or less minutes, 5 or less minutes, or 2 or less minutes.

A sample can be contacted by a fixation reagent for a period of time in a range of from 5 minutes to 24 hours, e.g., from 10 minutes to 20 hours, from 10 minutes to 18 hours, from 10 minutes to 12 hours, from 10 minutes to 8 hours, from 10 minutes to 6 hours, from 10 minutes to 4 hours, from 10 minutes to 2 hours, from 15 minutes to 20 hours, from 15 minutes to 18 hours, from 15 minutes to 12 hours, from 15 minutes to 8 hours, from 15 minutes to 6 hours, from 15 minutes to 4 hours, from 15 minutes to 2 hours, from 15 minutes to 1.5 hours, from 15 minutes to 1 hour, from 10 minutes to 30 minutes, from 15 minutes to 30 minutes, from 30 minutes to 2 hours, from 45 minutes to 1.5 hours, or from 55 minutes to 70 minutes.

A sample can be contacted by a fixation reagent at various temperatures, depending on the protocol and the reagent used. For example, in some instances a sample can be contacted by a fixation reagent at a temperature ranging from -22°C to 55°C, where specific ranges of interest include, but are not limited to 50 to 54°C, 40 to 44°C, 35 to 39°C, 28 to 32°C, 20 to 26°C, 0 to 6°C, and -18 to -22°C. In some instances, a sample can be contacted by a fixation reagent at a temperature of -20°C, 4°C, room temperature (22-25°C), 30°C, 37°C, 42°C, or 52°C.

Any convenient fixation reagent can be used. Common fixation reagents include crosslinking fixatives, precipitating fixatives, oxidizing fixatives, mercurials, and the like. Crosslinking fixatives chemically join two or more molecules by a covalent bond and a wide range of cross-linking reagents can be used. Examples of suitable cross-liking fixatives include but are not limited to aldehydes (e.g., formaldehyde, also commonly referred to as "paraformaldehyde" and "formalin"; glutaraldehyde; etc.), imidoesters, NHS (N- Hydroxysuccinimide) esters, and the like. Examples of suitable precipitating fixatives include but are not limited to alcohols (e.g., methanol, ethanol, etc.), acetone, acetic acid, etc. In some embodiments, the fixative is formaldehyde (i.e., paraformaldehyde or formalin). A suitable final concentration of formaldehyde in a fixation reagent is 0.1 to 10%, 1-8%, 1-4%, 1-2%, 3-5%, or 3.5-4.5%, including about 1.6% for 10 minutes. In some embodiments the sample is fixed in a final concentration of 4% formaldehyde (as diluted from a more concentrated stock solution, e.g., 38%, 37%, 36%, 20%, 18%, 16%, 14%, 10%, 8%, 6%, etc.). In some embodiments the sample is fixed in a final concentration of 10% formaldehyde. In some embodiments the sample is fixed in a final concentration of 1% formaldehyde. In some embodiments, the fixative is glutaraldehyde. A suitable concentration of glutaraldehyde in a fixation reagent is 0.1 to 1%. A fixation reagent can contain more than one fixative in any combination. For example, in some embodiments the sample is contacted with a fixation reagent containing both formaldehyde and glutaraldehyde.

The terms "permeabilization" or "permeabilize" as used herein refer to the process of rendering the cells (cell membranes etc.) of a sample permeable to experimental reagents such as nucleic acid probes, antibodies, chemical substrates, etc. Any convenient method and/or reagent for permeabilization can be used. Suitable permeabilization reagents include detergents (e.g., Saponin, Triton X-100, Tween-20, etc.), organic fixatives (e.g., acetone, methanol, ethanol, etc.), enzymes, etc. Detergents can be used at a range of concentrations. For example, 0.001%-1% detergent, 0.05%-0.5% detergent, or 0.1%-0.3% detergent can be used for permeabilization (e.g., 0.1 % Saponin, 0.2% tween-20, 0.1-0.3% triton X-100, etc.). In some embodiments methanol on ice for at least 10 minutes is used to permeabilize.

In some embodiments, the same solution can be used as the fixation reagent and the permeabilization reagent. For example, in some embodiments, the fixation reagent contains 0.1%-10% formaldehyde and 0.001%-1% saponin. In some embodiments, the fixation reagent contains 1% formaldehyde and 0.3% saponin.

A sample can be contacted by a permeabilization reagent for a wide range of times, which can depend on the temperature, the nature of the sample, and on the permeabilization reagent(s). For example, a sample can be contacted by a permeabilization reagent for 24 or more hours, 24 or less hours, 18 or less hours, 12 or less hours, 8 or less hours, 6 or less hours, 4 or less hours, 2 or less hours, 60 or less minutes, 45 or less minutes, 30 or less minutes, 25 or less minutes, 20 or less minutes, 15 or less minutes, 10 or less minutes, 5 or less minutes, or 2 or less minutes. A sample can be contacted by a permeabilization reagent at various temperatures, depending on the protocol and the reagent used. For example, in some instances a sample can be contacted by a permeabilization reagent at a temperature ranging from -82°C to 55°C, where specific ranges of interest include, but are not limited to: 50 to 54°C, 40 to 44°C, 35 to 39°C, 28 to 32°C, 20 to 26°C, 0 to 6°C, -18 to -22 °C, and -78 to -82°C. In some instances, a sample can be contacted by a permeabilization reagent at a temperature of -80°C, -20°C, 4°C, room temperature (22-25°C), 30°C, 37°C, 42°C, or 52°C.

In some embodiments, a sample is contacted with an enzymatic permeabilization reagent. Enzymatic permeabilization reagents that permeabilize a sample by partially degrading extracellular matrix or surface proteins that hinder the permeation of the sample by assay reagents. Contact with an enzymatic permeabilization reagent can take place at any point after fixation and prior to target detection. In some instances, the enzymatic permeabilization reagent is proteinase K, a commercially available enzyme. In such cases, the sample is contacted with proteinase K prior to contact with a post-fixation reagent. Proteinase K treatment (i.e., contact by proteinase K; also commonly referred to as "proteinase K digestion") can be performed over a range of times at a range of temperatures, over a range of enzyme concentrations that are empirically determined for each cell type or tissue type under investigation. For example, a sample can be contacted by proteinase K for 30 or less minutes, 25 or less minutes, 20 or less minutes, 15 or less minutes, 10 or less minutes, 5 or less minutes, or 2 or less minutes. A sample can be contacted by 1 µg/ml or less, 2 µg/m or less, 4 µg/ml or less, 8 µg/ml or less, 10 µg/ml or less, 20 µg/ml or less, 30 µg/ml or less, 50 µg/ml or less, or 100µg/ml or less proteinase K. A sample can be contacted by proteinase K at a temperature ranging from 2°C to 55°C, where specific ranges of interest include, but are not limited to: 50 to 54°C, 40 to 44°C, 35 to 39°C, 28 to 32°C, 20 to 26°C, and 0 to 6°C. In some instances, a sample can be contacted by proteinase K at a temperature of 4°C, room temperature (22-25°C), 30°C, 37°C, 42°C, or 52°C. In some embodiments, a sample is not contacted with an enzymatic permeabilization reagent. In some embodiments, a sample is not contacted with proteinase K. Contact of an intact tissue with at least a fixation reagent and a permeabilization reagent results in the production of a fixed and permeabilized tissue.

### Ligase

In some embodiments, the methods disclosed include adding ligase to ligate the second oligonucleotide and generate a closed nucleic acid circle. In some embodiments, the adding ligase includes adding DNA ligase. In alternative embodiments, the second oligonucleotide is provided as a closed nucleic acid circle, and the step of adding ligase is omitted. In certain embodiments, ligase is an enzyme that facilitates the sequencing of a target nucleic acid molecule.

The term "ligase" as used herein refers to an enzyme that is commonly used to join polynucleotides together or to join the ends of a single polynucleotide. Ligases include ATP-dependent double-strand polynucleotide ligases, NAD-dependent double-strand DNA or RNA ligases and single-strand polynucleotide ligases, for example any of the ligases described in EC 6.5.1 .1 (ATP-dependent ligases), EC 6.5.1 .2 (NAD+-dependent ligases), EC 6.5.1 .3 (RNA ligases). Specific examples of ligases include bacterial ligases such as E. coli DNA ligase and Taq DNA ligase, Ampligase^{®} thermostable DNA ligase (Epicentre^{®}Technologies Corp., part of Illumina^{®}, Madison, Wis.) and phage ligases such as T3 DNA ligase, T4 DNA ligase and T7 DNA ligase and mutants thereof.

### Rolling Circle Amplification

In some embodiments, the methods of the invention include the step of performing rolling circle amplification in the presence of a nucleic acid molecule, wherein the performing includes using the second oligonucleotide as a template and the first oligonucleotide as a primer for a polymerase to form one or more amplicons. In such embodiments, a single-stranded, circular polynucleotide template is formed by ligation of the second nucleotide, which circular polynucleotide includes a region that is complementary to the first oligonucleotide. Upon addition of a DNA polymerase in the presence of appropriate dNTP precursors and other cofactors, the first oligonucleotide is elongated by replication of multiple copies of the template. This amplification product can be readily detected by binding to a detection probe.

In some embodiments, only when a first oligonucleotide and second oligonucleotide hybridize to the same target nucleic acid molecule, the second oligonucleotide can be circularized and rolling-circle amplified to generate a cDNA nanoball (i.e., amplicon) containing multiple copies of the cDNA. The term "amplicon" refers to the amplified nucleic acid product of a PCR reaction or other nucleic acid amplification process. In some embodiments, amine-modified nucleotides are spiked into the rolling circle amplification reaction.

Techniques for rolling circle amplification are known in the art (see, e.g., Baner et al, Nucleic Acids Research, 26:5073-5078, 1998; Lizardi et al, Nature Genetics 19:226, 1998; Schweitzer et al. Proc. Natl Acad. Sci. USA 97:101 13- 1 19, 2000; Faruqi et al, BMC Genomics 2:4, 2000; Nallur et al, Nucl. Acids Res. 29:el 18, 2001 ; Dean et al. Genome Res. 1 1 :1095- 1099, 2001; Schweitzer et al, Nature Biotech. 20:359-365, 2002; U.S. Patent Nos. 6,054,274, 6,291,187, 6,323,009, 6,344,329 and 6,368,801).

In some embodiments the polymerase is phi29 DNA polymerase. In certain aspects, the nucleic acid molecule includes an amine-modified nucleotide. In such embodiments, the amine-modified nucleotide includes an acrylic acid N-hydroxysuccinimide moiety modification. Examples of other amine-modified nucleotides include, but are not limited to, a 5-Aminoallyl-dUTP moiety modification, a 5-Propargylamino-dCTP moiety modification, a N6-6-Aminohexyl-dATP moiety modification, or a 7-Deaza-7-Propargylamino-dATP moiety modification.

### Amplicon Embedding in a Tissue-hydrogel Setting

In some embodiments, the methods disclosed include embedding one or more amplicons in the presence of hydrogel subunits to form one or more hydrogel-embedded amplicons. The hydrogel-tissue chemistry described includes covalently attaching nucleic acids to *in situ* synthesized hydrogel for tissue clearing, enzyme diffusion, and multiple-cycles of sequencing. In some embodiments, to enable amplicon embedding in the tissue-hydrogel setting, amine-modified nucleotides are spiked into the rolling circle amplification reaction, functionalized with an acrylamide moiety using acrylic acid N-hydroxysuccinimide esters, and copolymerized with acrylamide monomers to form a hydrogel.

As used herein, the terms "hydrogel" or "hydrogel network" mean a network of polymer chains that are water-insoluble, sometimes found as a colloidal gel in which water is the dispersion medium. In other words, hydrogels are a class of polymeric materials that can absorb large amounts of water without dissolving. Hydrogels can contain over 99% water and may include natural or synthetic polymers, or a combination thereof. Hydrogels also possess a degree of flexibility very similar to natural tissue, due to their significant water content. A detailed description of suitable hydrogels may be found in published U.S. patent application 20100055733. As used herein, the terms "hydrogel subunits" or "hydrogel precursors" mean hydrophilic monomers, prepolymers, or polymers that can be crosslinked, or "polymerized", to form a three-dimensional (3D) hydrogel network. Without being bound by any scientific theory, it is believed that this fixation of the biological specimen in the presence of hydrogel subunits crosslinks the components of the specimen to the hydrogel subunits, thereby securing molecular components in place, preserving the tissue architecture and cell morphology.

In some embodiments, the embedding includes copolymerizing the one or more amplicons with acrylamide. As used herein, the term "copolymer" describes a polymer which contains more than one type of subunit. The term encompasses polymer which include two, three, four, five, or six types of subunits.

In certain aspects, the embedding includes clearing the one or more hydrogel-embedded amplicons wherein the target nucleic acid is substantially retained in the one or more hydrogel-embedded amplicons. In such embodiments, the clearing includes substantially removing a plurality of cellular components from the one or more hydrogel-embedded amplicons. In some other embodiments, the clearing includes substantially removing lipids from the one or more hydrogel-embedded amplicons. As used herein, the term "substantially" means that the original amount present in the sample before clearing has been reduced by approximately 70% or more, such as by 75% or more, such as by 80% or more, such as by 85% or more, such as by 90% or more, such as by 95% or more, such as by 99% or more, such as by 100%.

In some embodiments, clearing the hydrogel-embedded amplicons includes electrophoresing the specimen. In some embodiments, the amplicons are electrophoresed using a buffer solution that includes an ionic surfactant. In some embodiments, the ionic surfactant is sodium dodecyl sulfate (SDS). In some embodiments, the specimen is electrophoresed using a voltage ranging from about 10 to about 60 volts. In some embodiments, the specimen is electrophoresed for a period of time ranging from about 15 minutes up to about 10 days. In some embodiments, the methods further involve incubating the cleared specimen in a mounting medium that has a refractive index that matches that of the cleared tissue. In some embodiments, the mounting medium increases the optical clarity of the specimen. In some embodiments, the mounting medium includes glycerol.

### Sequencing with Error-correction by Dynamic Annealing and Ligation (SEDAL)

The methods disclosed herein include the step of contacting one or more hydrogel-embedded amplicons having the barcode sequence with a pair of primers under conditions to allow for ligation, wherein the pair of primers include a third oligonucleotide and a fourth oligonucleotide, wherein the ligation only occurs when both the third oligonucleotide and the fourth oligonucleotide ligate to the same amplicon. In some embodiments, the sequencing-by-ligation method is performed at room temperature for preservation of tissue morphology with low background noise and error reduction. In some embodiments, the contacting the one or more hydrogel-embedded amplicons includes eliminating error accumulation as sequencing proceeds.

In some embodiments, the contacting the one or more hydrogel-embedded amplicons occurs two times or more, including, but not limited to, e.g., three times or more, four times or more, five times or more, six times or more, or seven times or more. In certain embodiments, the contacting the one or more hydrogel-embedded amplicons occurs four times or more for thin tissue specimens. In other embodiments, the contacting the one or more hydrogel-embedded amplicons occurs six times or more for thick tissue specimens. In some embodiments, one or more amplicons can be contacted by a pair of primers for 24 or more hours, 24 or less hours, 18 or less hours, 12 or less hours, 8 or less hours, 6 or less hours, 4 or less hours, 2 or less hours, 60 or less minutes, 45 or less minutes, 30 or less minutes, 25 or less minutes, 20 or less minutes, 15 or less minutes, 10 or less minutes, 5 or less minutes, or 2 or less minutes.

Specimens prepared using the subject methods may be analyzed by any of a number of different types of microscopy, for example, optical microscopy (e.g. bright field, oblique illumination, dark field, phase contrast, differential interference contrast, interference reflection, epifluorescence, confocal microscopy, spinning disk confocal microscopy), light sheet microscopy, lattice light sheet microscopy, or light field microscopy, laser microscopy, electron microscopy, and scanning probe microscopy. In some aspects, a non-transitory computer readable medium transforms raw images acquired through microscopy of multiple rounds of *in situ* sequencing first into decoded gene identities and spatial locations and then analyzes the per-cell composition of gene expression.

### SEDAL Oligonucleotide Primers

In some embodiments, the methods disclosed include a third oligonucleotide and a fourth oligonucleotide. In certain aspects, the third oligonucleotide is configured to decode bases and the fourth oligonucleotide is configured to convert decoded bases into a signal. In some aspects, the signal is a fluorescent signal. In exemplary aspects, the contacting the one or more hydrogel-embedded amplicons having the barcode sequence with a pair of primers under conditions to allow for ligation involves each of the third oligonucleotide and the fourth oligonucleotide ligating to form a stable product for imaging only when a perfect match occurs. In certain aspects, the mismatch sensitivity of a ligase enzyme is used to determine the underlying sequence of the target nucleic acid molecule.

The term "perfectly matched", when used in reference to a duplex means that the polynucleotide and/or oligonucleotide strands making up the duplex form a double stranded structure with one another such that every nucleotide in each strand undergoes Watson-Crick base pairing with a nucleotide in the other strand. The term "duplex" includes, but is not limited to, the pairing of nucleoside analogs, such as deoxyinosine, nucleosides with 2-aminopurine bases, peptide nucleic acids (PNAs), and the like, that may be employed. A "mismatch" in a duplex between two oligonucleotides means that a pair of nucleotides in the duplex fails to undergo Watson-Crick bonding.

In some embodiments, the method includes a plurality of third oligonucleotides, including, but not limited to, 5 or more third oligonucleotides, e.g., 8 or more, 10 or more, 12 or more, 15 or more, 18 or more, 20 or more, 25 or more, 30 or more, 35 or more that hybridize to target nucleotide sequences. In some embodiments, a method of the present disclosure includes a plurality of third oligonucleotides, including, but not limited to, 15 or more third oligonucleotides, e.g., 20 or more, 30 or more, 40 or more, 50 or more, 60 or more, 70 or more, and up to 80 different first oligonucleotides that hybridize to 15 or more, e.g., 20 or more, 30 or more, 40 or more, 50 or more, 60 or more, 70 or more, and up to 80 different target nucleotide sequences. In some embodiments, the methods include a plurality of fourth oligonucleotides, including, but not limited to, 5 or more fourth oligonucleotides, e.g., 8 or more, 10 or more, 12 or more, 15 or more, 18 or more, 20 or more, 25 or more, 30 or more, 35 or more. In some embodiments, a method of the present disclosure includes a plurality of fourth oligonucleotides including, but not limited to, 15 or more fourth oligonucleotides, e.g., 20 or more, 30 or more, 40 or more, 50 or more, 60 or more, 70 or more, and up to 80 different first oligonucleotides that hybridize to 15 or more, e.g., 20 or more, 30 or more, 40 or more, 50 or more, 60 or more, 70 or more, and up to 80 different target nucleotide sequences. A plurality of oligonucleotide pairs can be used in a reaction, where one or more pairs specifically bind to each target nucleic acid. For example, two primer pairs can be used for one target nucleic acid in order to improve sensitivity and reduce variability. It is also of interest to detect a plurality of different target nucleic acids in a cell, e.g. detecting up to 2, up to 3, up to 4, up to 5, up to 6, up to 7, up to 8, up to 9, up to 10, up to 12, up to 15, up to 18, up to 20, up to 25, up to 30, up to 40 or more distinct target nucleic acids.

In certain embodiments, SEDAL involves a ligase with activity hindered by base mismatches, a third oligonucleotide, and a fourth oligonucleotide. The term "hindered" in this context refers to activity of a ligase that is reduced by approximately 20% or more, such as by 25% or more, such as by 50% or more, such as by 75% or more, such as by 90% or more, such as by 95% or more, such as by 99% or more, such as by 100%. In some embodiments, the third oligonucleotide has a length of 5-15 nucleotides, including, but not limited to, 5-13 nucleotides, 5-10 nucleotides, or 5-8 nucleotides. In some embodiments, the Tₘ of the third oligonucleotide is at room temperature (22-25°C). In some embodiments, the third oligonucleotide is degenerate, or partially thereof. In some embodiments, the fourth oligonucleotide has a length of 5-15 nucleotides, including, but not limited to, 5-13 nucleotides, 5-10 nucleotides, or 5-8 nucleotides. In some embodiments, the Tₘ of the fourth oligonucleotide is at room temperature (22- 25°C). After each cycle of SEDAL corresponding to a base readout, the fourth oligonucleotides may be stripped, which eliminates error accumulation as sequencing proceeds. In such embodiments, the fourth oligonucleotides are stripped by formamide.

In some embodiments, SEDAL involves the washing of the third oligonucleotide and the fourth oligonucleotide to remove unbound oligonucleotides, thereafter revealing a fluorescent product for imaging. In certain exemplary embodiments, a detectable label can be used to detect one or more nucleotides and/or oligonucleotides described herein. In certain embodiments, a detectable label can be used to detect the one or more amplicons. Examples of detectable markers include various radioactive moieties, enzymes, prosthetic groups, fluorescent markers, luminescent markers, bioluminescent markers, metal particles, protein-protein binding pairs, protein-antibody binding pairs and the like. Examples of fluorescent proteins include, but are not limited to, yellow fluorescent protein (YFP), green fluorescence protein (GFP), cyan fluorescence protein (CFP), umbelliferone, fluorescein, fluorescein isothiocyanate, rhodamine, dichlorotriazinylamine fluorescein, dansyl chloride, phycoerythrin and the like. Examples of bioluminescent markers include, but are not limited to, luciferase (e.g., bacterial, firefly, click beetle and the like), luciferin, aequorin and the like. Examples of enzyme systems having visually detectable signals include, but are not limited to, galactosidases, glucuronidases, phosphatases, peroxidases, cholinesterases and the like. Identifiable markers also include radioactive compounds such as ¹²⁵I, ³⁵S, ¹⁴C, or ³H. Identifiable markers are commercially available from a variety of sources.

Fluorescent labels and their attachment to nucleotides and/or oligonucleotides are described in many reviews, including Haugland, Handbook of Fluorescent Probes and Research Chemicals, Ninth Edition (Molecular Probes, Inc., Eugene, 2002); Keller and Manak, DNA Probes, 2nd Edition (Stockton Press, New York, 1993); Eckstein, editor, Oligonucleotides and Analogues: A Practical Approach (IRL Press, Oxford, 1991); and Wetmur, Critical Reviews in Biochemistry and Molecular Biology, 26:227-259 (1991). Particular methodologies applicable to the invention are disclosed in the following sample of references: U.S. Pat. Nos. 4,757,141, 5,151,507 and 5,091,519. In one aspect, one or more fluorescent dyes are used as labels for labeled target sequences, e.g., as disclosed by U.S. Pat. No. 5,188,934 (4,7-dichlorofluorescein dyes); U.S. Pat. No. 5,366,860 (spectrally resolvable rhodamine dyes); U.S. Pat. No. 5,847,162 (4,7-dichlororhodamine dyes); U.S. Pat. No. 4,318,846 (ether-substituted fluorescein dyes); U.S. Pat. No. 5,800,996 (energy transfer dyes); Lee et al.; U.S. Pat. No. 5,066,580 (xanthine dyes); U.S. Pat. No. 5,688,648 (energy transfer dyes); and the like. Labelling can also be carried out with quantum dots, as disclosed in the following patents and patent publications: U.S. Pat. Nos. 6,322,901, 6,576,291, 6,423,551, 6,251,303, 6,319,426, 6,426,513, 6,444,143, 5,990,479, 6,207,392, 2002/0045045 and 2003/0017264. As used herein, the term "fluorescent label" includes a signaling moiety that conveys information through the fluorescent absorption and/or emission properties of one or more molecules. Such fluorescent properties include fluorescence intensity, fluorescence lifetime, emission spectrum characteristics, energy transfer, and the like.

Commercially available fluorescent nucleotide analogues readily incorporated into nucleotide and/or oligonucleotide sequences include, but are not limited to, Cy3-dCTP, Cy3-dUTP, Cy5-dCTP, Cy5-dUTP (Amersham Biosciences, Piscataway, N.J.), fluorescein-12-dUTP, tetramethylrhodamine-6-dUTP, TEXAS RED^{™}-5-dUTP, CASCADE BLUE^{™}-7-dUTP, BODIPY TMFL-14-dUTP, BODIPY TMR-14-dUTP, BODIPY TMTR-14-dUTP, RHODAMINE GREEN^{™}-5-dUTP, OREGON GREENR^{™} 488-5-dUTP, TEXAS RED^{™}-12-dUTP, BODIPY^{™} 630/650-14-dUTP, BODIPY^{™} 650/665-14-dUTP, ALEXA FLUOR^{™} 488-5-dUTP, ALEXA FLUOR^{™} 532-5-dUTP, ALEXA FLUOR^{™} 568-5-dUTP, ALEXA FLUOR^{™} 594-5-dUTP, ALEXA FLUOR^{™} 546-14-dUTP, fluorescein-12-UTP, tetramethylrhodamine-6-UTP, TEXAS RED^{™}-5-UTP, mCherry, CASCADE BLUE^{™}-7-UTP, BODIPY^{™} FL-14-UTP, BODIPY TMR-14-UTP, BODIPY^{™} TR-14-UTP, RHODAMINE GREEN^{™}-5-UTP, ALEXA FLUOR^{™} 488-5-UTP, LEXA FLUOR^{™} 546-14-UTP (Molecular Probes, Inc. Eugene, Oreg.) and the like. Protocols are known in the art for custom synthesis of nucleotides having other fluorophores (See, Henegariu et al. (2000) Nature Biotechnol. 18:345).

Other fluorophores available for post-synthetic attachment include, but are not limited to, ALEXA FLUOR^{™} 350, ALEXA FLUOR^{™} 532, ALEXA FLUOR^{™} 546, ALEXA FLUOR^{™} 568, ALEXA FLUOR^{™} 594, ALEXA FLUOR^{™} 647, BODIPY 493/503, BODIPY FL, BODIPY R6G, BODIPY 530/550, BODIPY TMR, BODIPY 558/568, BODIPY 558/568, BODIPY 564/570, BODIPY 576/589, BODIPY 581/591, BODIPY 630/650, BODIPY 650/665, Cascade Blue, Cascade Yellow, Dansyl, lissamine rhodamine B, Marina Blue, Oregon Green 488, Oregon Green 514, Pacific Blue, rhodamine 6G, rhodamine green, rhodamine red, tetramethyl rhodamine, Texas Red (available from Molecular Probes, Inc., Eugene, Oreg.), Cy2, Cy3.5, Cy5.5, Cy7 (Amersham Biosciences, Piscataway, N.J.) and the like. FRET tandem fluorophores may also be used, including, but not limited to, PerCP-Cy5.5, PE-Cy5, PE-Cy5.5, PE-Cy7, PE-Texas Red, APC-Cy7, PE-Alexa dyes (610, 647, 680), APC-Alexa dyes and the like.

Metallic silver or gold particles may be used to enhance signal from fluorescently labeled nucleotide and/or oligonucleotide sequences (Lakowicz et al. (2003) Bio Techniques 34:62).

Biotin, or a derivative thereof, may also be used as a label on a nucleotide and/or an oligonucleotide sequence, and subsequently bound by a detectably labeled avidin/streptavidin derivative (e.g. phycoerythrin-conjugated streptavidin), or a detectably labeled anti-biotin antibody. Digoxigenin may be incorporated as a label and subsequently bound by a detectably labeled anti-digoxigenin antibody (e.g. fluoresceinated anti-digoxigenin). An aminoallyl-dUTP residue may be incorporated into an oligonucleotide sequence and subsequently coupled to an N-hydroxy succinimide (NHS) derivatized fluorescent dye. In general, any member of a conjugate pair may be incorporated into a detection oligonucleotide provided that a detectably labeled conjugate partner can be bound to permit detection. As used herein, the term antibody refers to an antibody molecule of any class, or any sub-fragment thereof, such as an Fab.

Other suitable labels for an oligonucleotide sequence may include fluorescein (FAM), digoxigenin, dinitrophenol (DNP), dansyl, biotin, bromodeoxyuridine (BrdU), hexahistidine (6×His), phosphor-amino acids (e.g. P-tyr, P-ser, P-thr) and the like. In one embodiment the following hapten/antibody pairs are used for detection, in which each of the antibodies is derivatized with a detectable label: biotin/α-biotin, digoxigenin/α-digoxigenin, dinitrophenol (DNP)/α-DNP, 5-Carboxyfluorescein (FAM)/α-FAM.

In certain exemplary embodiments, a nucleotide and/or an oligonucleotide sequence can be indirectly labeled, especially with a hapten that is then bound by a capture agent, e.g., as disclosed in U.S. Pat. Nos. 5,344,757, 5,702,888, 5,354,657, 5,198,537 and 4,849,336, PCT publication WO 91/17160 and the like. Many different hapten-capture agent pairs are available for use. Exemplary haptens include, but are not limited to, biotin, des-biotin and other derivatives, dinitrophenol, dansyl, fluorescein, CY5, digoxigenin and the like. For biotin, a capture agent may be avidin, streptavidin, or antibodies. Antibodies may be used as capture agents for the other haptens (many dye-antibody pairs being commercially available, e.g., Molecular Probes, Eugene, Oreg.).

### Cells

Methods disclosed herein include a method for *in situ* gene sequencing of a target nucleic acid in a cell in an intact tissue. In certain embodiments, the cell is present in a population of cells. In certain other embodiments, the population of cells includes a plurality of cell types including, but not limited to, excitatory neurons, inhibitory neurons, and non-neuronal cells. Cells for use in the assays of the invention can be an organism, a single cell type derived from an organism, or can be a mixture of cell types. Included are naturally occurring cells and cell populations, genetically engineered cell lines, cells derived from transgenic animals, etc. Virtually any cell type and size can be accommodated. Suitable cells include bacterial, fungal, plant and animal cells. In one embodiment of the invention, the cells are mammalian cells, e.g. complex cell populations such as naturally occurring tissues, for example blood, liver, pancreas, neural tissue, bone marrow, skin, and the like. Some tissues may be disrupted into a monodisperse suspension. Alternatively, the cells may be a cultured population, e.g. a culture derived from a complex population, a culture derived from a single cell type where the cells have differentiated into multiple lineages, or where the cells are responding differentially to stimulus, and the like.

Cell types that can find use in the subject invention include stem and progenitor cells, e.g. embryonic stem cells, hematopoietic stem cells, mesenchymal stem cells, neural crest cells, etc., endothelial cells, muscle cells, myocardial, smooth and skeletal muscle cells, mesenchymal cells, epithelial cells; hematopoietic cells, such as lymphocytes, including T-cells, such as Th1 T cells, Th2 T cells, ThO T cells, cytotoxic T cells; B cells, pre- B cells, etc.; monocytes; dendritic cells; neutrophils; and macrophages; natural killer cells; mast cells, etc.; adipocytes, cells involved with particular organs, such as thymus, endocrine glands, pancreas, brain, such as neurons, glia, astrocytes, dendrocytes, etc. and genetically modified cells thereof. Hematopoietic cells may be associated with inflammatory processes, autoimmune diseases, etc., endothelial cells, smooth muscle cells, myocardial cells, etc. may be associated with cardiovascular diseases; almost any type of cell may be associated with neoplasias, such as sarcomas, carcinomas and lymphomas; liver diseases with hepatic cells; kidney diseases with kidney cells; etc.

The cells may also be transformed or neoplastic cells of different types, e.g. carcinomas of different cell origins, lymphomas of different cell types, etc. The American Type Culture Collection (Manassas, VA) has collected and makes available over 4,000 cell lines from over 150 different species, over 950 cancer cell lines including 700 human cancer cell lines. The National Cancer Institute has compiled clinical, biochemical and molecular data from a large panel of human tumor cell lines, these are available from ATCC or the NCI (Phelps et al. (1996) Journal of Cellular Biochemistry Supplement 24:32-91). Included are different cell lines derived spontaneously, or selected for desired growth or response characteristics from an individual cell line; and may include multiple cell lines derived from a similar tumor type but from distinct patients or sites.

Cells may be non-adherent, e.g. blood cells including monocytes, T cells, B-cells; tumor cells, etc., or adherent cells, e.g. epithelial cells, endothelial cells, neural cells, etc. In order to profile adherent cells, they may be dissociated from the substrate that they are adhered to, and from other cells, in a manner that maintains their ability to recognize and bind to probe molecules.

Such cells can be acquired from an individual using, e.g., a draw, a lavage, a wash, surgical dissection etc., from a variety of tissues, e.g., blood, marrow, a solid tissue (e.g., a solid tumor), ascites, by a variety of techniques that are known in the art. Cells may be obtained from fixed or unfixed, fresh or frozen, whole or disaggregated samples. Disaggregation of tissue may occur either mechanically or enzymatically using known techniques.

### Imaging

The methods disclosed include imaging the one or more hydrogel-embedded amplicons using any of a number of different types of microscopy, e.g., confocal microscopy, two-photon microscopy, light-field microscopy, intact tissue expansion microscopy, and/or CLARITY^{™}-optimized light sheet microscopy (COLM).

Bright field microscopy is the simplest of all the optical microscopy techniques. Sample illumination is via transmitted white light, i.e. illuminated from below and observed from above. Limitations include low contrast of most biological samples and low apparent resolution due to the blur of out of focus material. The simplicity of the technique and the minimal sample preparation required are significant advantages.

In oblique illumination microscopy, the specimen is illuminated from the side. This gives the image a 3-dimensional appearance and can highlight otherwise invisible features. A more recent technique based on this method is Hoffmann's modulation contrast, a system found on inverted microscopes for use in cell culture. Though oblique illumination suffers from the same limitations as bright field microscopy (low contrast of many biological samples; low apparent resolution due to out of focus objects), it may highlight otherwise invisible structures.

Dark field microscopy is a technique for improving the contrast of unstained, transparent specimens. Dark field illumination uses a carefully aligned light source to minimize the quantity of directly-transmitted (unscattered) light entering the image plane, collecting only the light scattered by the sample. Dark field can dramatically improve image contrast (especially of transparent objects) while requiring little equipment setup or sample preparation. However, the technique suffers from low light intensity in final image of many biological samples, and continues to be affected by low apparent resolution.

Phase contrast is an optical microscopy illumination technique that converts phase shifts in light passing through a transparent specimen to brightness changes in the image. In other words, phase contrast shows differences in refractive index as difference in contrast. The phase shifts themselves are invisible to the human eye, but become visible when they are shown as brightness changes.

In differential interference contrast (DIC) microscopy, differences in optical density will show up as differences in relief. The system consists of a special prism (Nomarski prism, Wollaston prism) in the condenser that splits light in an ordinary and an extraordinary beam. The spatial difference between the two beams is minimal (less than the maximum resolution of the objective). After passage through the specimen, the beams are reunited by a similar prism in the objective. In a homogeneous specimen, there is no difference between the two beams, and no contrast is being generated. However, near a refractive boundary (e.g. a nucleus within the cytoplasm), the difference between the ordinary and the extraordinary beam will generate a relief in the image. Differential interference contrast requires a polarized light source to function; two polarizing filters have to be fitted in the light path, one below the condenser (the polarizer), and the other above the objective (the analyzer).

Another microscopic technique using interference is interference reflection microscopy (also known as reflected interference contrast, or RIC). It is used to examine the adhesion of cells to a glass surface, using polarized light of a narrow range of wavelengths to be reflected whenever there is an interface between two substances with different refractive indices. Whenever a cell is attached to the glass surface, reflected light from the glass and that from the attached cell will interfere. If there is no cell attached to the glass, there will be no interference.

A fluorescence microscope is an optical microscope that uses fluorescence and phosphorescence instead of, or in addition to, reflection and absorption to study properties of organic or inorganic substances. In fluorescence microscopy, a sample is illuminated with light of a wavelength which excites fluorescence in the sample. The fluoresced light, which is usually at a longer wavelength than the illumination, is then imaged through a microscope objective. Two filters may be used in this technique; an illumination (or excitation) filter which ensures the illumination is near monochromatic and at the correct wavelength, and a second emission (or barrier) filter which ensures none of the excitation light source reaches the detector. Alternatively, these functions may both be accomplished by a single dichroic filter. The "fluorescence microscope" refers to any microscope that uses fluorescence to generate an image, whether it is a more simple set up like an epifluorescence microscope, or a more complicated design such as a confocal microscope, which uses optical sectioning to get better resolution of the fluorescent image.

In some embodiments, an anti-oxidant compound is included in the washing and imaging buffers (i.e., "anti-fade buffers") to reduce photobleaching during fluorescence imaging. Exemplary anti-oxidants include, without limitation, propyl-gallate, tertiary butylhydroquinone, butylated hydroxyanisole, butylated hydroxytoluene, glutathione, ascorbic acid, and tocopherols. Such anti-oxidants have an antifade effect on fluorophores. That is, the anti-oxidant reduces photobleaching during tiling, greatly enhances the SNR of sensitive fluorophores, and enables higher SNR imaging of thicker samples. For a fixed exposure time, including an anti-oxidant increases the SNR by increasing the concentration of the non-bleached fluorophore during exposure to light. Including an anti-oxidant also removes the diminishing returns of longer exposure times (caused by the limited fluorophore lifetime before photobleaching), providing for increased SNR by allowing increased exposure times.

In addition, fluorophore cleavage from probes (as opposed to probe stripping) is used in the CISITS method to eliminate signal from round to round. Including a disulfide linkage between the fluorophore and an oligonucleotide probe enables cleavage of the fluorophore from the oligonucleotide probe in a reducing environment. Following fluorescence imaging of the sequencing round, a reducing agent is added to reduce the disulfide bond connecting the fluorophore to the oligonucleotide, and subsequent washing steps remove the diffusive fluorescent signal before performing another round. The use of SEDAL sequencing with thiol-based chemical cleavage in CISITS combines the specificity and SNR advantages of SEDAL sequencing with the robustness and rapidity of round-to-round cycling from fluorophore cleavage.

Confocal microscopy uses point illumination and a pinhole in an optically conjugate plane in front of the detector to eliminate out-of-focus signal. As only light produced by fluorescence very close to the focal plane can be detected, the image's optical resolution, particularly in the sample depth direction, is much better than that of wide-field microscopes. However, as much of the light from sample fluorescence is blocked at the pinhole, this increased resolution is at the cost of decreased signal intensity - so long exposures are often required. As only one point in the sample is illuminated at a time, 2D or 3D imaging requires scanning over a regular raster (i.e., a rectangular pattern of parallel scanning lines) in the specimen. The achievable thickness of the focal plane is defined mostly by the wavelength of the used light divided by the numerical aperture of the objective lens, but also by the optical properties of the specimen. The thin optical sectioning possibly makes these types of microscopes particularly good at 3D imaging and surface profiling of samples. COLM provides an alternative microscopy for fast 3D imaging of large clarified samples. COLM interrogates large immunoassayed tissues, permits increased speed of acquisition and results in a higher quality of generated data.

In single plane illumination microscopy (SPIM), also known as light sheet microscopy, only the fluorophores in the focal plane of the detection objective lens are illuminated. The light sheet is a beam that is collimated in one and focused in the other direction. Since no fluorophores are excited outside the detectors' focal plane, the method also provides intrinsic optical sectioning. Moreover, when compared to conventional microscopy, light sheet methods exhibit reduced photobleaching and lower phototoxicity, and often enable far more scans per specimen. By rotating the specimen, the technique can image virtually any plane with multiple views obtained from different angles. For every angle, however, only a relatively shallow section of the specimen is imaged with high resolution, whereas deeper regions appear increasingly blurred.

Super-resolution microscopy is a form of light microscopy. Due to the diffraction of light, the resolution of conventional light microscopy is limited as stated by Ernst Abbe in 1873. A good approximation of the resolution attainable is the FWHM (full width at half-maximum) of the point spread function, and a precise widefield microscope with high numerical aperture and visible light usually reaches a resolution of ~250 nm. Super-resolution techniques allow the capture of images with a higher resolution than the diffraction limit. They fall into two broad categories, "true" super-resolution techniques, which capture information contained in evanescent waves, and "functional" super-resolution techniques, which use experimental techniques and known limitations on the matter being imaged to reconstruct a super-resolution image.

Laser microscopy uses laser illumination sources in various forms of microscopy. For instance, laser microscopy focused on biological applications uses ultrashort pulse lasers, or femtosecond lasers, in a number of techniques including nonlinear microscopy, saturation microscopy, and multiphoton fluorescence microscopy such as two-photon excitation microscopy (a fluorescence imaging technique that allows imaging of living tissue up to a very high depth, e.g. one millimeter)

In electron microscopy (EM), a beam of electrons is used to illuminate a specimen and produce a magnified image. An electron microscope has greater resolving power than a light-powered optical microscope because electrons have wavelengths about 100,000 times shorter than visible light (photons). They can achieve better than 50 pm resolution and magnifications of up to about 10,000,000x whereas ordinary, non-confocal light microscopes are limited by diffraction to about 200 nm resolution and useful magnifications below 2000x. The electron microscope uses electrostatic and electromagnetic "lenses" to control the electron beam and focus it to form an image. These lenses are analogous to but different from the glass lenses of an optical microscope that form a magnified image by focusing light on or through the specimen. Electron microscopes are used to observe a wide range of biological and inorganic specimens including microorganisms, cells, large molecules, biopsy samples, metals, and crystals. Industrially, the electron microscope is often used for quality control and failure analysis. Examples of electron microscopy include Transmission electron microscopy (TEM), Scanning electron microscopy (SEM), reflection electron microscopy (REM), Scanning transmission electron microscopy (STEM) and low-voltage electron microscopy (LVEM).

Scanning probe microscopy (SPM) is a branch of microscopy that forms images of surfaces using a physical probe that scans the specimen. An image of the surface is obtained by mechanically moving the probe in a raster scan of the specimen, line by line, and recording the probe-surface interaction as a function of position. Examples of SPM include atomic force microscopy (ATM), ballistic electron emission microscopy (BEEM), chemical force microscopy (CFM), conductive atomic force microscopy (C-AFM), electrochemical scanning tunneling microscope (ECSTM), electrostatic force microscopy (EFM), fluidic force microscope (FluidFM), force modulation microscopy (FMM), feature-oriented scanning probe microscopy (FOSPM), kelvin probe force microscopy (KPFM), magnetic force microscopy (MFM), magnetic resonance force microscopy (MRFM), near-field scanning optical microscopy (NSOM) (or SNOM, scanning near-field optical microscopy, SNOM, Piezoresponse Force Microscopy (PFM), PSTM, photon scanning tunneling microscopy (PSTM), PTMS, photothermal microspectroscopy/microscopy (PTMS), SCM, scanning capacitance microscopy (SCM), SECM, scanning electrochemical microscopy (SECM), SGM, scanning gate microscopy (SGM), SHPM, scanning Hall probe microscopy (SHPM), SICM, scanning ion-conductance microscopy (SICM), SPSM spin polarized scanning tunneling microscopy (SPSM), SSRM, scanning spreading resistance microscopy (SSRM), SThM, scanning thermal microscopy (SThM), STM, scanning tunneling microscopy (STM), STP, scanning tunneling potentiometry (STP), SVM, scanning voltage microscopy (SVM), and synchrotron x-ray scanning tunneling microscopy (SXSTM).

Intact tissue expansion microscopy (exM) enables imaging of thick preserve specimens with roughly 70 nm lateral resolution. Using ExM the optical diffraction limit is circumvented by physically expanding a biological specimen before imaging, thus bringing sub-diffraction limited structures into the size range viewable by a conventional diffraction-limited microscope. ExM can image biological specimens at the voxel rates of a diffraction limited microscope, but with the voxel sizes of a super-resolution microscope. Expanded samples are transparent, and index-matched to water, as the expanded material is >99% water. Techniques of expansion microscopy are known in the art, e.g., as disclosed in Gao et al., Q&A: Expansion Microscopy, BMC Biol. 2017; 15:50.

### Screening Methods

The methods disclosed herein also provide for a method of screening a candidate agent to determine whether the candidate agent modulates gene expression of a nucleic acid in a cell in an intact tissue, the method including: (a) contacting a fixed and permeabilized intact tissue with at least a pair of oligonucleotide primers under conditions to allow for specific hybridization, wherein the pair of primers include a first oligonucleotide and a second oligonucleotide; wherein each of the first oligonucleotide and the second oligonucleotide includes a first complementarity region, a second complementarity region, and a third complementarity region; wherein the second oligonucleotide further includes a barcode sequence; wherein the first complementarity region of the first oligonucleotide is complementary to a first portion of the target nucleic acid, wherein the second complementarity region of the first oligonucleotide is complementary to the first complementarity region of the second oligonucleotide, wherein the third complementarity region of the first oligonucleotide is complementary to the third complementarity region of the second oligonucleotide, wherein the second complementary region of the second oligonucleotide is complementary to a second portion of the target nucleic acid, and wherein the first complementarity region of the first oligonucleotide is adjacent to the second complementarity region of the second oligonucleotide; (b) adding ligase to ligate the second oligonucleotide and generate a closed nucleic acid circle; (c) performing rolling circle amplification in the presence of a nucleic acid molecule, wherein the performing includes using the second oligonucleotide as a template and the first oligonucleotide as a primer for a polymerase to form one or more amplicons; (d) embedding the one or more amplicons in the presence of hydrogel subunits to form one or more hydrogel-embedded amplicons; (e) contacting the one or more hydrogel-embedded amplicons having the barcode sequence with a pair of primers under conditions to allow for ligation, wherein the pair of primers include a third oligonucleotide and a fourth oligonucleotide, wherein the ligation only occurs when both the third oligonucleotide and the fourth oligonucleotide ligate to the same amplicon; (f) reiterating step (e); (g) imaging the one or more hydrogel-embedded amplicons to determine in situ gene sequencing of the target nucleic acid in the cell in the intact tissue; and (h) detecting the level of gene expression of the target nucleic acid, wherein an alteration in the level of expression of the target nucleic acid in the presence of the at least one candidate agent relative to the level of expression of the target nucleic acid in the absence of the at least one candidate agent indicates that the at least one candidate agent modulates gene expression of the nucleic acid in the cell in the intact tissue. Such screening methods include the steps of CISITS provided herein.

In some aspects, the detecting includes performing flow cytometry; sequencing; probe binding and electrochemical detection; pH alteration; catalysis induced by enzymes bound to DNA tags; quantum entanglement; Raman spectroscopy; terahertz wave technology; and/or scanning electron microscopy. In certain aspects, the flow cytometry is mass cytometry or fluorescence-activated flow cytometry. In some other aspects, the detecting includes performing microscopy, scanning mass spectrometry or other imaging techniques described herein. In such aspects, the detecting includes determining a signal, e.g. a fluorescent signal.

By "test agent," "candidate agent," and grammatical equivalents herein, which terms are used interchangeably herein, is meant any molecule (e.g. proteins (which herein includes proteins, polypeptides, and peptides), small (i.e., 5-1000 Da, 100-750 Da, 200-500 Da, or less than 500 Da in size), or organic or inorganic molecules, polysaccharides, polynucleotides, etc.) which are to be tested for activity in a subject assay.

A variety of different candidate agents may be screened by the above methods. Candidate agents encompass numerous chemical classes, e.g., small organic compounds having a molecular weight of more than 50 daltons (e.g., at least about 50 Da, at least about 100 Da, at least about 150 Da, at least about 200 Da, at least about 250 Da, or at least about 500 Da) and less than about 20,000 daltons, less than about 10,000 daltons, less than about 5,000 daltons, or less than about 2,500 daltons. For example, in some embodiments, a suitable candidate agent is an organic compound having a molecular weight in a range of from about 500 Da to about 20,000 Da, e.g., from about 500 Da to about 1000 Da, from about 1000 Da to about 2000 Da, from about 2000 Da to about 2500 Da, from about 2500 Da to about 5000 Da, from about 5000 Da to about 10,000 Da, or from about 10,000 Da to about 20,000 Da.

Candidate agents can include functional groups necessary for structural interaction with proteins, e.g., hydrogen bonding, and can include at least an amine, carbonyl, hydroxyl or carboxyl group, or at least two of the functional chemical groups. The candidate agents can include cyclical carbon or heterocyclic structures and/or aromatic or polyaromatic structures substituted with one or more of the above functional groups. Candidate agents are also found among biomolecules including peptides, saccharides, fatty acids, steroids, purines, pyrimidines, derivatives, structural analogs or combinations thereof.

Candidate agents are obtained from a wide variety of sources including libraries of synthetic or natural compounds. For example, numerous means are available for random and directed synthesis of a wide variety of organic compounds and biomolecules, including expression of randomized oligonucleotides and oligopeptides. Alternatively, libraries of natural compounds in the form of bacterial, fungal, plant and animal extracts are available or readily produced. Additionally, natural or synthetically produced libraries and compounds are readily modified through conventional chemical, physical and biochemical means, and may be used to produce combinatorial libraries. Known pharmacological agents may be subjected to directed or random chemical modifications, such as acylation, alkylation, esterification, amidification, etc. to produce structural analogs. Moreover, screening may be directed to known pharmacologically active compounds and chemical analogs thereof, or to new agents with unknown properties such as those created through rational drug design.

In one embodiment, candidate modulators are synthetic compounds. Any number of techniques is available for the random and directed synthesis of a wide variety of organic compounds and biomolecules, including expression of randomized oligonucleotides. See for example WO 94/24314, which discusses methods for generating new compounds, including random chemistry methods as well as enzymatic methods.

In another embodiment, the candidate agents are provided as libraries of natural compounds in the form of bacterial, fungal, plant and animal extracts that are available or readily produced. Additionally, natural or synthetically produced libraries and compounds are readily modified through conventional chemical, physical and biochemical means. Known pharmacological agents may be subjected to directed or random chemical modifications, including enzymatic modifications, to produce structural analogs.

In one embodiment, candidate agents include proteins (including antibodies, antibody fragments (i.e., a fragment containing an antigen-binding region, single chain antibodies, and the like), nucleic acids, and chemical moieties. In one embodiment, the candidate agents are naturally occurring proteins or fragments of naturally occurring proteins. Thus, for example, cellular extracts containing proteins, or random or directed digests of proteinaceous cellular extracts, may be tested. In this way libraries of prokaryotic and eukaryotic proteins may be made for screening. Other embodiments include libraries of bacterial, fungal, viral, and mammalian proteins (e.g., human proteins).

In one embodiment, the candidate agents are organic moieties. In this embodiment, as is generally described in WO 94/243 14, candidate agents are synthesized from a series of substrates that can be chemically modified. "Chemically modified" herein includes traditional chemical reactions as well as enzymatic reactions. These substrates generally include, but are not limited to, alkyl groups (including alkanes, alkenes, alkynes and heteroalkyl), aryl groups (including arenes and heteroaryl), alcohols, ethers, amines, aldehydes, ketones, acids, esters, amides, cyclic compounds, heterocyclic compounds (including purines, pyrimidines, benzodiazepins, beta-lactams, tetracylines, cephalosporins, and carbohydrates), steroids (including estrogens, androgens, cortisone, ecodysone, etc.), alkaloids (including ergots, vinca, curare, pyrollizdine, and mitomycines), organometallic compounds, hetero-atom bearing compounds, amino acids, and nucleosides. Chemical (including enzymatic) reactions may be done on the moieties to form new substrates or candidate agents which can then be tested using the present invention.

### Devices and Systems

Also included are devices for performing aspects of the CISITS methods. The subject devices may include, for example, sample chambers, imaging chambers, electrophoresis apparatus, flow chambers, microscopes, needles, tubing, and pumps.

The present disclosure also provides systems for performing the subject methods. Systems may include one or more of the modules described herein, e.g. a power supply, a refrigeration unit, waste, a heating unit, a pump, etc. Systems may also include any of the reagents described herein, e.g. imaging buffer, wash buffer, strip buffer, Nissl and DAPI solutions. Systems in accordance with certain embodiments may also include a microscope and/or related imaging equipment, e.g., camera components, digital imaging components and/or image capturing equipment, computer processors configured to collect images according to one or more user inputs, and the like.

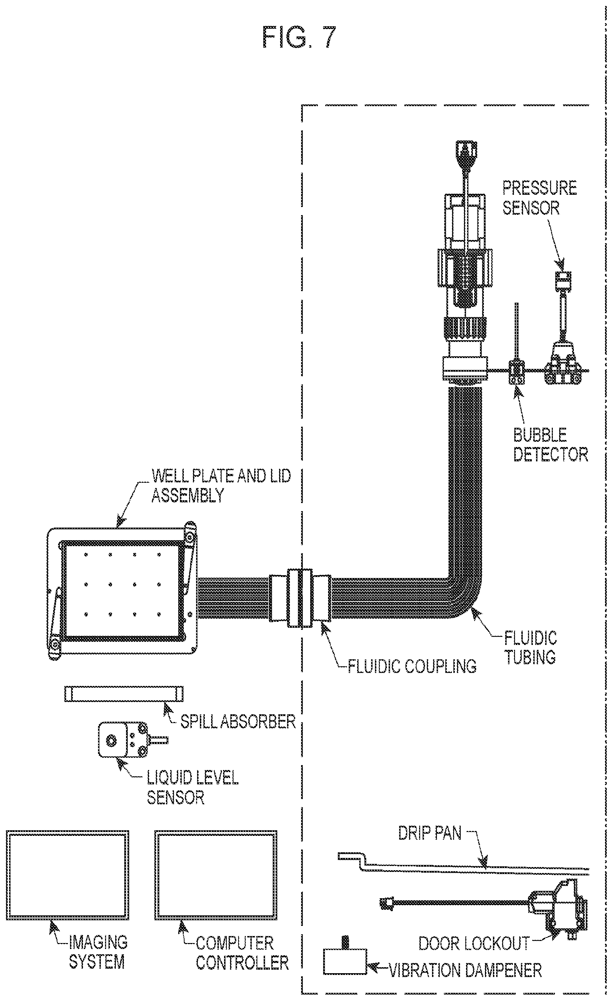

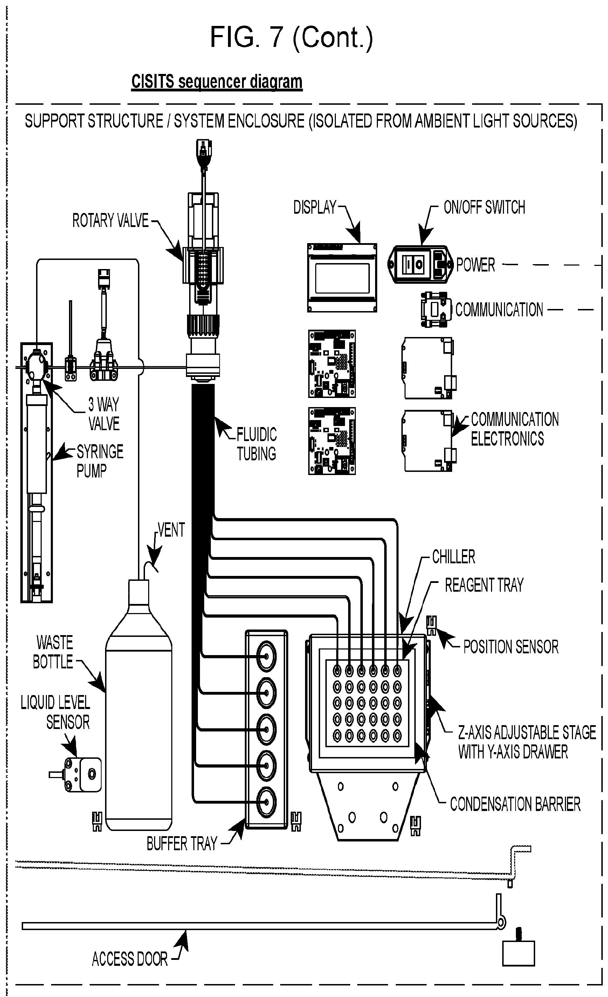

An exemplary fluidic system for performing the CISITS methods described herein is shown in FIG. 7. The systems described herein include a fluidic device having an unpressurized sample chamber comprising a lid with an interface tube, wherein a first end of the interface tube is held by the lid, and a second end of the interface tube is positioned above a tissue sample in the sample chamber. In some embodiments, the sample chamber is a well of a multiwell plate (e.g., 6, 12, or 24-well plate) or a slide chamber. The interface tube is preferably positioned sufficiently close to a bottom edge of the sample chamber such that liquid within the sample chamber remains in contact with the bottom of the interface tube as long as liquid remains in the sample chamber. Sufficient space should be left between the bottom of the sample interface tube and the sample chamber that liquid added to the sample does not pressurize the interface tube but instead spreads over and bathes the sample. In some embodiments, the interface tube is placed at a polar angle relative to the tissue sample plane. A fluidic line is connected to the first end of the interface tube such that fluid flows through the fluidic line into the interface tube and flows out of the second end of the interface tube onto the tissue sample in the sample chamber. A cryostat may be included in the fluidic system to maintain the sample chamber at a cryogenic temperature. The fluidic system also comprises an imaging system, a pump, and a processor unit configured to perform the CISITS methods described herein.

In some embodiments, the system enables the automation of CISITS, a process described herein as including, but not limited to, repeated rounds of hybridization of probes with DNA embedded in a gel, ligation of fluorescently labeled oligonucleotides onto these probes, washing off the excess probes, imaging, and fluorophore cleavage from probes before performing the next round of sequencing. In some embodiments, the system may allow for continual operation. In some embodiments, the system includes an imaging chamber for flowing sequencing chemicals involved in *in situ* DNA sequencing over a sample. In some embodiments, the system of fluidics and pumps control sequencing chemical delivery to the sample.

Buffers may be added/removed/recirculated/replaced by the use of the one or more ports and optionally, tubing, pumps, valves, or any other suitable fluid handling and/or fluid manipulation equipment, for example, tubing that is removably attached or permanently attached to one or more components of a device. For example, a first tube having a first and second end may be attached to a first port and a second tube having a first and second end may be attached to a second port, where the first end of the first tube is attached to the first port and the second end of the first tube is operably linked to a receptacle, e.g. a cooling unit, heating unit, filtration unit, waste receptacle, etc.; and the first end of the second tube is attached to the second port and the second end of the second tube is operably linked to a receptacle, e.g. a cooling unit, beaker on ice, filtration unit, waste receptacle, etc.

In some embodiments, a multiway selector valve is interfaced with the pump. The multiway selector valve is used to switch which fluidic line is currently open to allow different modes of operation. The pump can be used to pull or to push a volume of fluid. For example, a syringe pump can be used to pull a desired amount of reagent into the pump via a series of selector valves. In some embodiments, the multiport selector valve is coupled to a tray of reagents or buffers to allow the pump to pull selected reagents or buffers into the system. The fluidic system may further comprise an adjustable stage supporting the reagent tray and a position sensor that allows positioning of the adjustable stage to allow selection of a desired reagent from the reagent tray, which is fluidically connected to the multiway selector valve. A chiller may be included in the fluidic system to maintain a desired temperature of the reagents. The pump can also be used to pull reagents from a sample well via the interface tube. In addition, the pump can be used to push a volume, e.g., to a sample well via the interface tube, or to a waste container. Alternatively, the pump can push a volume in reverse back to the reagent bank (which may be used for cleaning purposes).

It is preferable to use fresh reagents in performing the CISITS method, particularly for SEDAL sequencing, rolling circle amplification, and ligation. In some embodiments, disposable reagent trays comprising reagents for performing the CISITS method are used in the fluidic system to make the use of fresh reagents convenient for the user.

In some embodiments, the system further comprises means for cryosectioning a tissue sample and placing a tissue section flat on a surface inside the sample chamber. For example, the sample chamber (e.g., well of a multiwell plate or a slide chamber) can be kept cold in a cryostat, along with a thin-walled metal ring. After a section is collected, the metal ring is pressed down onto the section such that the edges of the ring evenly contact the frozen cutting media surrounding the section. The ring and flat section can be transferred to the sample chamber. The metal ring facilitates placing the section flat onto a surface inside the sample chamber, and the warmth from a finger can be used to separate the section from the ring and adhere it to the sample chamber surface.

In some embodiments, the system is enclosed in a container that blocks ambient light. The container may contain a number of doors and drawers for accessing various internal components of the system. For example, the system may contain a drawer for placing a sample into the system and removing it, and a drawer for placing the reagents into the system and removing them. Other components of the enclosure may provide access for service professionals to alter, upgrade, or fix internal parts of the system. The enclosure may also be acoustic dampening.

In some embodiments, the system includes a non-transitory computer-readable storage medium that has instructions, which when executed by the processor unit, cause the processor unit to control the delivery of chemicals and synchronize this process with a microscope. In some embodiments, the non-transitory computer-readable storage medium includes instructions, which when executed by the processor unit, cause the processor unit to measure an optical signal.

### Utility

The devices, methods, and systems herein find a number of uses in the art such as in biomedical research and/or clinical diagnostics. For example, in biomedical research, applications include, but are not limited to, spatially resolved gene expression analysis for fundamental biology or drug screening. In clinical diagnostics, applications include, but are not limited to, detecting gene markers such as disease, immune responses, bacterial or viral DNA/RNA for patient samples. Examples of advantages of the methods described herein include efficiency, where it takes merely 3 or 4 days to obtain final data from a raw sample, providing speeds much faster than existing microarray or sequencing technology; highly multiplexed (up to 1000 genes); single-cell and single-molecule sensitivity; preserved tissue morphology; and/or high signal-to-noise ratio with low error rates.

In certain aspects, CISITS may be applied to the study of molecular-defined cell types and activity-regulated gene expression in mouse visual cortex, and to be scalable to larger 3D tissue blocks to visualize short- and long- range spatial organization of cortical neurons on a volumetric scale not previously accessible. In some embodiments, the methods disclosed herein may be adapted to image DNA-conjugated antibodies for highly multiplexed protein detection.

The devices, methods, and systems of the invention can also be generalized to study a number of heterogeneous cell populations in diverse tissues. Without being bound by any scientific theory, the brain poses special challenges well suited to CISITS analysis. For example, the polymorphic activity-regulated gene (ARG) expression observed across different cell types is likely to depend on both intrinsic cell-biological properties (such as signal transduction pathway-component expression), and on extrinsic properties such as neural circuit anatomy that routes external sensory information to different cells (here in visual cortex). In such cases, in situ transcriptomics exemplified by CISITS can effectively link imaging-based molecular information with anatomical and activity information, thus elucidating brain function and dysfunction.

The devices, methods, and systems disclosed herein enable cellular components, e.g. lipids that normally provide structural support but that hinder visualization of subcellular proteins and molecules to be removed while preserving the 3-dimensional architecture of the cells and tissue because the sample is crosslinked to a hydrogel that physically supports the ultrastructure of the tissue. This removal renders the interior of biological specimen substantially permeable to light and/or macromolecules, allowing the interior of the specimen, e.g. cells and subcellular structures, to be microscopically visualized without time-consuming and disruptive sectioning of the tissue. The procedure is also more rapid than procedures commonly used in the art, as clearance and permeabilization, typically performed in separate steps, may be combined in a single step of removing cellular components. Additionally, the specimen can be iteratively stained, unstained, and re-stained with other reagents for comprehensive analysis. Further functionalization with the polymerizable acrylamide moiety enables amplicons to be covalently anchored within the polyacrylamide network at multiple sites.

In one example, the subject devices, methods, and systems may be employed to evaluate, diagnose or monitor a disease. "Diagnosis" as used herein generally includes a prediction of a subject's susceptibility to a disease or disorder, determination as to whether a subject is presently affected by a disease or disorder, prognosis of a subject affected by a disease or disorder (e.g., identification of cancerous states, stages of cancer, likelihood that a patient will die from the cancer), prediction of a subject's responsiveness to treatment for a disease or disorder (e.g., a positive response, a negative response, no response at all to, e.g., allogeneic hematopoietic stem cell transplantation, chemotherapy, radiation therapy, antibody therapy, small molecule compound therapy) and use of therametrics (e.g., monitoring a subject's condition to provide information as to the effect or efficacy of therapy). For example, a biopsy may be prepared from a cancerous tissue and microscopically analyzed to determine the type of cancer, the extent to which the cancer has developed, whether the cancer will be responsive to therapeutic intervention, etc.

The subject devices, methods, and systems also provide a useful technique for screening candidate therapeutic agents for their effect on a tissue or a disease. For example, a subject, e.g. a mouse, rat, dog, primate, human, etc. may be contacted with a candidate agent, an organ or a biopsy thereof may be prepared by the subject methods, and the prepared specimen microscopically analyzed for one or more cellular or tissue parameters. Parameters are quantifiable components of cells or tissues, particularly components that can be accurately measured, desirably in a high throughput system. A parameter can be any cell component or cell product including cell surface determinant, receptor, protein or conformational or posttranslational modification thereof, lipid, carbohydrate, organic or inorganic molecule, nucleic acid, e.g. mRNA, DNA, etc. or a portion derived from such a cell component or combinations thereof. While most parameters will provide a quantitative readout, in some instances a semi-quantitative or qualitative result will be acceptable. Readouts may include a single determined value, or may include mean, median value or the variance, etc. Characteristically a range of parameter readout values will be obtained for each parameter from a multiplicity of the same assays. Variability is expected and a range of values for each of the set of test parameters will be obtained using standard statistical methods with a common statistical method used to provide single values. Thus, for example, one such method may include detecting cellular viability, tissue vascularization, the presence of immune cell infiltrates, efficacy in altering the progression of the disease, etc. In some embodiments, the screen includes comparing the analyzed parameter(s) to those from a control, or reference, sample, e.g., a specimen similarly prepared from a subject not contacted with the candidate agent. Candidate agents of interest for screening include known and unknown compounds that encompass numerous chemical classes, primarily organic molecules, which may include organometallic molecules, inorganic molecules, genetic sequences, etc. Candidate agents of interest for screening also include nucleic acids, for example, nucleic acids that encode siRNA, shRNA, antisense molecules, or miRNA, or nucleic acids that encode polypeptides. An important aspect of the invention is to evaluate candidate drugs, including toxicity testing; and the like. Evaluations of tissue samples using the subject methods may include, e.g., genetic, transcriptomic, genomic, proteomic, and/or metabolomics analyses.

The subject devices, methods, and systems may also be used to visualize the distribution of genetically encoded markers in whole tissue at subcellular resolution, for example, chromosomal abnormalities (inversions, duplications, translocations, etc.), loss of genetic heterozygosity, the presence of gene alleles indicative of a predisposition towards disease or good health, likelihood of responsiveness to therapy, ancestry, and the like. Such detection may be used in, for example, diagnosing and monitoring disease as, e.g., described above, in personalized medicine, and in studying paternity.

A database of analytic information can be compiled. These databases may include results from known cell types, references from the analysis of cells treated under particular conditions, and the like. A data matrix may be generated, where each point of the data matrix corresponds to a readout from a cell, where data for each cell may include readouts from multiple labels. The readout may be a mean, median or the variance or other statistically or mathematically derived value associated with the measurement. The output readout information may be further refined by direct comparison with the corresponding reference readout. The absolute values obtained for each output under identical conditions will display a variability that is inherent in live biological systems and also reflects individual cellular variability as well as the variability inherent between individuals.

### EXPERIMENTAL

The following examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. Efforts have been made to ensure accuracy with respect to numbers used (e.g. amounts, temperature, *etc*.) but some experimental errors and deviations should be accounted for. Unless indicated otherwise, parts are parts by weight, molecular weight is weight average molecular weight, temperature is in degrees Centigrade, and pressure is at or near atmospheric.

The present invention has been described in terms of particular embodiments found or proposed by the present inventor to comprise preferred modes for the practice of the invention. It will be appreciated by those of skill in the art that, in light of the present disclosure, numerous modifications and changes can be made in the particular embodiments exemplified without departing from the intended scope of the invention. For example, due to codon redundancy, changes can be made in the underlying DNA sequence without affecting the protein sequence. Moreover, due to biological functional equivalency considerations, changes can be made in protein structure without affecting the biological action in kind or amount. All such modifications are intended to be included within the scope of the appended claims.

### Example 1

### New Methods for Intact-Tissue Sequencing Chemistry and Process

### Introduction

STARmap is a sequencing process in which target nucleic acids are selectively labeled with barcodes, which are amplified and affixed into a hydrogel before subsequent multi-round readout by ligation. The original publication of STARmap demonstrated the usage of combinatorial sequencing on sample sizes as large as 0.0034 cubic millimeters and the usage of sequential sequencing on sample sizes as large as 0.238 cubic millimeters. Collecting these data required considerable human effort; the read-out stage of sequencing alone required multiple continuous days of effort for a single sample. An entire adult mouse brain is roughly 400 cubic millimeters, or more than 110,000x the volume of the previously demonstrated combinatorial sequencing sample. As intact-tissue sequencing approaches scale to increasing volumes and throughputs, maximizing their robustness (to prevent the compounding accumulation of error) and enhancing their parallelism and throughput is of critical importance. This is especially the case as these techniques move out of the lab and into clinical and industrial workflows.

The new method we describe here targets several different robustness and throughput aspects of the previous STARmap technique, including both the sequencing chemistry and the automated execution of sequencing. We term this new method "Clinical and Industrial Scale Intact Tissue Sequencing or CISITS. The chemistry aspects of the new method focuses on increasing the signal-to-noise ratio (SNR) of labeled molecules, eliminating carryover signal across rounds, achieving reliable efficiencies and chemistry success across the protocol, and enabling combinatorial sequencing in thick tissues; the new automation methods include a fluidic system in which the sample chamber is no longer pressurized, a combination of system integration methods for high system robustness, and methods for parallelization across samples; CISITS software control methods manage the automated fluidic system and orchestrate a robust fluidic exchange process while preserving the sequencing workflow in the event of a failure; and CISITS integrations with microscope hardware provide systems integration simplifying and accelerating the user interaction and enabling large field-of-view acquisitions for enhanced throughput.

### CISITS

### (1) New methods for high sequencing SNR

In the original version of STARmap, amplicons are labeled during sequencing by coupling fluorescently labeled oligos to the appropriate amplicons such that they remain attached at room temperature after washing. Both the post-ligation washes and imaging use a "washing and imaging" buffer, which was formulated with 2X SSC, 10% formamide, and water. Imaging in this buffer suffered from photobleaching and loss of signal from the fluorophores with longer exposure times. This is problematic both in early rounds of sequencing (when signal is highest) due to image tiling for large fields of view, which increases the total light power delivered to the edges of the fields of view and decreases the SNR and detectability of individual amplicons. It is especially problematic in later rounds of sequencing, which suffer a characteristic decline in SNR due to the nature of the SEDAL sequencing chemistry. At later rounds, especially for longer wavelength fluorophores, poor SNR begins to compromise the ability to extract high quality reads from molecules, decreasing the overall yield of sequenced molecules, since successful sequencing requires a complete barcode read.

We altered the washing and imaging buffer formulation to include anti-oxidant compounds, such as N-propyl gallate. For instance, a new formulation of the washing and imaging buffer is 2X SSC, water, and 10% of 20% N-propyl gallate dissolved in formamide, for a final concentration of 10% formamide and 2% N-propyl gallate. Inclusion of anti-oxidant compounds in the washing and imaging buffer functions as an antifade for the fluorophores. This reduces photobleaching during tiling, greatly enhances the SNR of sensitive fluorophores, and enables higher SNR imaging of thicker samples. For a fixed exposure time, including anti-oxidant compounds increases the SNR by increasing the concentration of non-bleached fluorophore per dot across an exposure. Including anti-oxidant also removes the diminishing returns effect of longer exposure times (caused by a limited fluorophore lifetime before bleaching), enabling linear increases in SNR by increasing exposure times as necessary.

### (2) Improvements to round-to-round signal separation.

In the sequential readout approach to STARmap sequencing, barcoded amplicons from individual genes are probed only on a single round, such that a given color channel conveys information strictly about a distinct gene of interest per round. This sequential readout approach is used in thicker tissue sections, in which it is more difficult to control amplicon size and maintain the separation of amplicons across rounds; or in samples in which fewer numbers of genes need to be read out and emphasis is placed more on volumetric throughput, which is obtained by using lower magnitude objectives and camera binning, for instance 4x4 binning of pixels per image. The success of this sequential approach requires that signal from a given color channel is totally eliminated from round to round. If it is not, then, for instance, remaining signal from a highly expressed gene in one round may be mistaken for signal from a lower expressed gene in a subsequent round.

We solved this problem by increasing the concentration of formamide from 60% to 80%, and increasing the length and number of formamide stripping cycles on every round. Although, this increased the efficiency of signal removal and completely removed signal for all genes; its efficiency is subject to variations in room temperature, and can add considerable extra time per sample. The additional formamide exposure can damage the quality of the sample across time and rounds of sequencing.

To more completely eliminate signal from round to round, another aspect of CISITS involves combined SEDAL-based sequencing readout with fluorophore cleavage (as opposed to probe stripping). Including a thiol linkage between the fluorophore and the rest of the oligo enables cleavage of the fluorophore in a reducing environment (which has been used in other in situ sequencing approaches), such as TCEP solution. However, in STARmap SEDAL sequencing, high SNR labeling of amplicons occurs during a ligation reaction; ligation buffers contain DTT, a reducing agent, which we found to dramatically reduce the signal from thiol-containing fluorescent oligos.

CISITS reformulates the SEDAL sequencing/ligation buffer to remove the DTT component. We found that we could attach thiol-containing fluorophore-labeled oligos to target amplicons with little difference in specificity or SNR from non-thiol-containing fluorescent oligos. The removal of DTT additionally allows the thiol-containing fluorophore probes to remain stable at 4°C in ligation mix for at least 24 hours with only minimal loss of subsequent labeling efficacy, facilitating automation. Following imaging of the sequencing round, adding buffer containing the reducing agent TCEP for 30 minutes completely removes punctate fluorescent signal, and short subsequent PBSTw washes removes diffusive fluorescent signal.

This aspect of the new method has the advantages of robust signal removal, expedited round time through the reduction in signal-removal/wash times, and should especially convey constant tissue robustness, enabling greater total number of rounds without loss of sample integrity. The use of SEDAL sequencing with thiol-based chemical cleavage in CISITS combines the specificity and SNR advantages of SEDAL sequencing with the robustness and rapidity of round-to-round cycling from fluorophore cleavage.

### (3) New methods for robustness in the CISITS protocol prior to and during sequencing.

### Tissue section collection

In intact tissue combinatorial sequencing, fresh frozen tissue sections are collected by cryosectioning and placing sections onto fresh, bind-silane coated wells or slides. In order to facilitate high throughput, parallel processing of samples, it is preferable to collect samples in a multi-well plate, such as a 24-well plate. However, it is difficult to transfer frozen sections onto silane-coated plate bottoms without tissue disruption, folding, or tearing, which can preclude subsequent data collection and analysis. Moreover, since the tissue collection and preparation prior to sequence read-out should be highly parallelizable across samples, optimal sample collection is essential. The STARmap method does not detail a process for robust sample collection, and standard approaches have a very high failure rate per section, resulting in many lost sections and inefficient use of space on plates.

In order to reliably place frozen sections flat onto surfaces used in downstream processing, we devised a new method as part of CISITS in which the sample collection wells are kept cold in a cryostat, along with a small, 8 or 10mm diameter, thin-walled metal ring. After a section is collected, the metal ring is pressed down onto the section such that the edges of the ring evenly contact the frozen cutting media surrounding the section. The ring and flat section can then be picked up using forceps and transferred to the appropriate surface, which is warmed just prior on the underside by a finger. The metal ring facilitates placing the section exactly flat onto the silane surface, and the warmth from a finger separates the section from the ring and adheres it to the well. This new method greatly reliably ensures section integrity and throughput.

### CISITS formulation of Acrylic Acid NHS Ester Buffer

The previous formulation in the STARmap method of AA NHS Ester buffer was not pH buffered. In the new CISITS method, including 20 mM MOPS pH 7.7 in the AA NHS Ester buffer pH stabilizes the buffer at a more optimal pH for the AA NHS Ester, making the subsequent NHS Ester reaction reliable and efficient.

### CISITS protocols for reaction robustness.

Several aspects of the sequencing chemistry have been found to require fresh reagents for robust high throughput use across multiple samples and multiple days of sequencing, which is especially important for scalable and automated sequencing. In the new CISITS method:

Polymerization II, RCA, and ligation mixtures must be made fresh for use in the CISITS process prior to sequencing.

Ligation mixtures for SEDAL sequencing rounds can be premixed and stored at 4°C for at least 24 hours. Signal is optimal when making SEDAL ligation round mixtures fresh for each 24 hours of sequencing, provided that they are stored at 4°C. During automated sequencing, additional SEDAL reagent for rounds >4 may be added fresh to the sequencing device after several rounds of operation have completed.

TCEP buffer is optimally used when made fresh before each sequencing run, but maintains efficiency across sequencing cycles.

### (4) New methods for combinatorial labeling of nucleic acids in thicker tissues.

A major limitation to the throughput of the STARmap method is the inability to perform combinatorial sequencing on thicker tissues. This limitation stems from several sources. First, because enzymatic reactions are required to ligate SNAIL probes and to amplify ligated probes using rolling circle amplification (RCA), enzyme penetration into the sample and subsequent efficiency can be highly limiting. An additional limitation is uniformity in the control of size of RCA amplicons. Due to the difficult diffusive properties of thicker tissues, obtaining uniform amplicons of suitable size for multi-round is not possible under the previous STARmap method. Moreover, the cost of obtaining probes suitably modified for direct incorporation into a hydrogel (before ligation and amplification) can be prohibitive for large numbers of genes.

In the new method, we devised a number of approaches for breaking through these limitations to enable large-scale sequencing of tissue. These new methods provide several different avenues for obtaining thick-section combinatorial and high throughput sequencing.

In the first instance of this new CITSIT method, probes containing proper end modifications such as acrydite are used for hybridization, as in the STARmap method. They may be purchased individually, modified in lab, or obtained as a pool of oligos. Following hybridization, a hydrogel is formed prior to digestion, ligation and RCA. Following digestion and ligation, amplification is performed using a new method to achieve ample, uniform ligation and amplification efficiencies across the sample. First, the RCA mixture is formulated including the enzyme and buffer but excluding dNTPs, thus preventing any amplification from proceeding. This mixture is added to the ligated sample and allowed to diffuse in to the sample until uniformity, for instance over the course of two days. By removing the dNTPs, the slow enzyme diffusion process can proceed to equilibrium. Following the addition of the RCA mixture without dNTPs and its equilibration, an RCA mixture with an excess concentration dNTPs is added to the sample. Because dNTPs are small molecules and have a high concentration gradient outside the sample, they will rapidly equilibrate into the sample at room temperature. Following, for example, 30 minutes of equilibration with the RCA mixture containing dNTPs at room temperature, the temperature is increased to the optimal temperature for amplification and amplification is allowed to proceed for several hours, producing uniform and appropriately sized amplicons for subsequent multiround combinatorial sequencing.

In a second instance of the new method, endogenous RNAs are first attached to a hydrogel, prior to hybridization. This is accomplished by compounds capable of polymerizing into the hydrogel that mediate the interaction with endogenous RNA either covalently, through attachment chemistry such as labelX, or by attachment to the 5' methyl RNA mRNA cap, or by highly stable hybridization to the polyA tail, or by short degenerate probes that individually can be displaced by subsequent target hybridization, but collectively serve to hold the endogenous RNA in the hydrogel with sufficient long term stability. Subsequent to hydrogel formation with RNAs embedded, the sample may be aggressively digested and permeabilized, such that a clear hydrogel is obtained containing mostly endogenous RNAs. This resulting hydrogel has superior diffusive properties compared to non-digested tissue. Probe hybridization, ligation, and RCA can then proceed with moderate incubation times, though methods for uniform RCA amplification described in the first instance ensure compatibility with downstream combinatorial sequencing. Following RCA, the amplicons are linked into the hydrogel following the previously described approach in the thin section STARmap method for long term stability before proceeding to combinatorial, multi-round sequencing.

Due to the order-of-magnitude increased thickness of these samples compared to the thin-section methods, incorporation of N-propyl gallate or other antioxidant compounds in the washing and imaging buffer is essential to prevent bleaching as the large depth of the sample in Z is imaged.

### New automated fluidic system hardware methods for CISITS

Previous automated methods for STARmap sequencing consisted of a fluidic system in which sequencing reagents are pushed and pulled across a sample in a pressurized, air-free flow cell. Correct and robust functioning of this flow-cell-based system requires the elimination of bubbles from the fluidic lines, properly managed reagent flow in the flow cell such that reagents are fully exchanged on each step of the sequencing, adequate priming of the systems to ensure the correct amount of each reagent is loaded, and proper sealing of the flow cell when connecting to the fluidics. Failures in any of these requirements at any point in the multiday sequencing can lead to sequencing failure. Meeting these requirements is complicated by the heterogenous shape and size of the sample on the flow cell. Additionally, a basic incompatibility exists between the STARmap methods for sample preparation prior to sequencing, and the previously described methods for automated STARmap sequencing in a flow cell. Namely, existing STARmap methods allow for parallel preparation of many samples adhered to individual wells of a multiwell plate, but do not specify any method for transferring the hybridized, ligated, and amplified, and gellated sample into a flow cell, which is difficult due to the adhered nature of the sample to the sample preparation plate bottom. No methods have been described for performing the STARmap sample preparation methods on a flow cell from the start, which would suffer from evaporation and liquid management issues, in addition to the loss of ease of parallelization across distinct samples. Finally, previous STARmap automation methods described sequencing of only a single sample (or flow cell) at a time, severely limiting the throughput of the method. We have developed new methods for solving many of these robustness and throughput issues of previous methods.

### CISITS fluidic system

### New methods for sample interfacing

The CISITS fluidic system adds and removes sequencing reagents from a sample, however, unlike previous methods, this new method uses an unpressurized sample chamber, which can accommodate air over the top of the sample. Relaxing the requirement for reagent flow over the sample in favor of simply bathing the sample in reagent greatly improves system robustness and simplicity while greatly facilitating parallelization of automated sequencing across different sample chambers.

In this system, one or multiple samples sits in a sample chamber, which may be a glass-bottom well of a multiwell plate (for optimal compatibility with the CISITS method prior to sequencing) or any glass bottom chamber in which air in the chamber permitted to exchange with room air, relieving flow pressure. In one instance, the sample prepared using the CISITS method in a well of a 6, 12, or 24-well multiwell glass-bottom plate. The fluidic system is coupled to the sample via a custom well-plate lid, which holds a stiff fluidic tube (such as a metal tube) just above the sample near the bottom edge of the sample well (interface tube). A fluidic line is coupled with this tube such that fluid passes through the fluidic line, into the interface tube, and onto the sample in the well, bathing it. To remove fluid from the sample, fluid is sucked out of the sample well via the interface tube. Different custom lids for the CISITS fluidic system are designed to meet the format of different sample chambers, including a variety of different format multiwell plates or multiple arrangements of slide chambers. In the case of multiwell plates, the system may couple with multiple different sample wells simultaneously, providing access to different sample wells (to be sequenced in series) or alternatively providing parallel sequencing capabilities across wells (described below).

The custom CISITS lids that couple the fluidic system to the sample are not sealed onto the sample chamber or plate, such that they are easily placed on or removed at any point, and such that they allow for air exchange. However, the fluidic interface tube is precisely positioned within the lids, such that liquid within the sample chamber remains in contact (via cohesion and adhesion) with the bottom of the tube as long as liquid remains in the chamber. This ensures that all or nearly all reagent is removed from the sample well on each reagent removal step, yielding a robust automation process. Sufficient space is left between the bottom of the sample interface tube and the sample chamber that liquid added to the sample does not pressurize the interface tube but instead spreads over and bathes the sample. Sample interface tubes may be placed at polar angles relative to the plane of the sample, such that many tubes may fit onto a container with many different sample chambers, such that each chamber has an interface tube. Alternatively, the custom CISITS lid may allow the user to place single or multiple interface tubes at some desired sample chambers such that the tube is reliably guided into an exact position that proffers robust fluid exchange. These custom lids may be machined or 3D printed to specification, and may be consumables or reused.

### New methods for serial fluid flow through reagent selection, sample and disposal

The fluidic system for CISITS is centered on a syringe pump, which pulls a desired amount of reagent into the pump via a series of selector valves. In the case of single sample operation, the pump in this new method has several operation modes. It can pull reagent in from a multiport selector valve coupled to a bank of reagents, or it can pull reagent in from the sample well via the interface tube. Once the pump has pulled in some volume, it can push the volume either to the sample well via the interface tube, or it can push the volume to a waste container, or it can push the volume in reverse back to the reagent bank (which may be used for cleaning purposes). These different operation modes are mediated by a multiway selector valve (for example, 3-way) interfaced with the pump, which switches which fluidic line is currently open. Thus, an example of a normal mode of operation for serial sequencing is: open only the pump selector to the reagent selector line, open the desired multiport selector line for the reagent of interest, and pull a selected reagent from the reagent bank through the multiport selector and into the pump; allow equilibration to occur to control for varying flow times due to different liquid properties; open the pump selector only towards the sample well and raise the syringe pump, pushing reagent through the sample fluidic line and the interface tube to bathe the sample; repeat this process pulling a small volume of air into the pump instead of reagent and push it to the sample, flushing the sample fluidic line of any remaining reagent and delivering the full desired reagent volume to the sample; wait for any amount of incubation time as part of the CISITS sequencing process; pull the pump down and suck liquid from sample via the interface tube back into the pump and allow for equilibration; open only the pump selector port to the waste, and raise the pump to push its contents into the waste; repeat this liquid removal process sufficient times to totally remove a desired volume from the sample. This can be considered a reagent addition, incubation, and removal cycle in normal operation of the CISITS method. The cycle is repeated either for the same or different reagents as the sample proceeds through the CISITS sequencing process. This cycling process of the fluidic system briefly leaves the sample without liquid, unlike a flow cell process. However, because the CISITS method uses tissue imbedded in a hydrogel, the sample does not dry out on this timescale and does not need to be constantly immersed. Moreover, because the sample is adhered to the bottom of the chamber, and is imaged by an inverted microscope, no air is able to move between the bottom of the well and the sample, thus, air bubbles are not an issue in this method.

This non-pressurized sample chamber method for the fluidic system enables various robustness methods, such as the aforementioned movement of air through the system after reagent addition to ensure complete reagent addition to the sample. It additionally allows a greater volume of reagent than is available to be pulled by the pump, bringing in additional air volume to the pump but ensuring that any limiting amount of reagent is completely drawn up from the reagent container. The fluidic system in this method can bypass the sample by pulling liquid into the pump and pushing it directly to the waste container, which expedites the washing process; water, dilute bleach, or other cleaning solution can be drawn into the pump to clean it and push directly to waste. Alternatively, water or other cleaning solution could be pushed into a reagent container if desired, for instance to clean the reagent fluidic lines.

### New methods for parallel fluid flow

This new CISITS method in which the sample chamber is not pressurized greatly facilitates parallelization of fluid delivery across multiple different sample chambers. In this new method, additional selector valves mediate the fluidic line from the pump to the samples. These selector valves may be open singly (allowing for addressable access to the different sample wells) or have multiple open simultaneously, allowing for fluid to be delivered in parallel by the pump to multiple samples. By coupling the output fluidic lines from this sample selector valve to each different sample interface tube and sample, parallel sequencing with CISITS can be achieved, offering substantial throughput in sequencing multiple samples. For instance, instead of a serial sequencing process in which a single sample is sequenced at a time, taking approximately 48 hours each, many samples could be sequenced simultaneously or in an interleaved fashion, such that the image acquisition system becomes the limiting process instead of sequencing cycles or incubation times. For example, for a single sequencing round (out of 7 or more), reagent addition, incubation, and removal collectively may consume 80% of the round completion time, while imaging consumes 20%. Operating in parallel across samples over 5 samples may offer a 4x throughput speedup (with 1x time devoted to imaging all 5 samples on each round); alternatively, operating in a staggered or interleaved fashion may offer a nearly 5x speedup (less one round time for staggering), as following the first round the image acquisition could be run nearly constantly. Thus, this new method results in a near linear increase in the throughput of intact tissue sequencing with the number of parallel sequenced samples up to the number of samples at which the image acquisition system becomes limiting.

### New methods for reagent management system

In the previous STARmap automated sequencing method, reagents were kept chilled via distinct sample tubes in a cold block or in tubes at room temperature; each reagent was directly coupled to the fluidic system, requiring the user to switch reagents in between runs by directly coupling and uncoupling the fluidic system from the reagents.

In the new method, a reagent management system abstracts this process for the user, and simply provides, for instance, a disposable reagent tray which can be simply placed into the fluidic device. The device places the proper portion of this reagent tray onto an internal cold block and automatically couples the fluidic lines into the reagents. The reagent containers or wells within the reagent tray have sufficient heat transfer properties to allow coupling with the chiller unit. Following a CISITS run/completion of the automatic fluidics operation, the tray can be disposed and a new tray can be used for a new run. The user fills the appropriate reagents into labeled areas of the reagent tray before placing it into the fluidics system. The reagent tray may vary in the volume contained for different reagents, depending on the throughput of the system and whether samples are being sequenced by the CISITS method in parallel or serially.

To ensure freshness of the sequencing reagents, the tray may be optionally removed part way through a sequencing run in order to place the next several rounds of freshly mixed SEDAL reagents.

This new method for reagent management simplifies and expedites the initialization of a CISITS run and provides a robust process that prevents the user from disrupting the normal operation of the system fluidics.

### New methods for enclosure of the CISITS sequencing system

The whole sequencing system is enclosed by a container, which blocks light while providing appropriate doors and drawers for accessing various internal components of the system. Some of the drawers are intended for normal use of the system, such as a drawer for placing a sample into the system and removing it, and a drawer for placing the reagents into the system and removing them. Other components of the enclosure provide easier access for service professionals to alter, upgrade, or fix internal parts of the system. The enclosure can include acoustic dampening.

### New methods for robust operation of the fluidic system

The CISITS automated sequencing system contains multiple methods for ensuring robustness and a low failure rate of automatic operation. These methods include, for example: detectors for fluid flow through the sample fluidic line, to ensure fluid delivery; an absorbent material surrounding the imaging objective and electrodes to detect reagent spillage from the sample container, whose activity can halt sequencing to prevent catastrophic damage; checks to ensure the chiller has reached the appropriate temperature before reagents are loaded; checks to detect proper loading of the CISITS reagents / tray into the system; regular system washes, automatically performed at the end of each sequencing run, and intermittent washes performed on the system before runs in the case that the system has not been recently used, in order to prevent any jamming of the fluidic lines by salt or other material build up. In the washes performed automatically at the end of sequencing, dilute bleach or other cleaning solution is moved into the pump and then to waste, and is followed by multiple rounds of washes with water to completely remove the bleach or cleaning solution. After automated procedures have finished, the system requests the user to place a wash sample container that matches the format previously used by the user, so that bleach or cleaning solution and water can be passed through the sample fluidic lines and tubes in order to clean them without damaging the sequenced sample, or this wash can be performed with the users consent on the sample container automatically. Finally, air is passed through the system to remove remaining liquid in the fluidic lines. Intermediate washes can be started by the user using a wash sample plate in between sequencing runs, provided that a wash reagent tray has been placed into the device with wash buffer and/or dilute bleach, and water. These wash cycles push wash liquid through both the pump, sample fluidic lines, and the reagent fluidic lines, remove the wash liquid to waste, perform multiple cycles of washes with water, and then flush the system with air.

### New methods for system integrations of fluidics and microscopy enabling CISITS

The new CISITS method requires an image acquisition system or microscope capable of rapidly acquiring images of the sample across the sample depth and with a resolution as high as sub-micron for proper dot separation during combinatorial sequencing. Multiple different microscopes and styles of microscopy can be compatible with the CISITS fluidic system, including confocal microscopy, spinning disk confocal microscopy, light sheet microscopy, lattice light sheet microscopy, and light field microscopy, depending on whether combinatorial sequencing (greater requirement for resolution such that individual amplicons are resolved) or sequential sequencing (lesser requirement for resolution) with CISITS is being performed.

In one instance, spinning disk confocal microscopy is coupled with the automated CISITS fluidic system. The CISITS fluidic system communicates via software with the microscope system in order to synchronize or interleave image acquisition with the fluidic process. This communication may be serial, through a REST API, through TTL triggers, or by other software methods.

In one instance, the microscope is a consumer product, communicates by software with the CISITS sequencing management, and is coupled to the automated fluidic system mechanically simply by the user's placement of the sample container onto the microscope stage. The user users the microscope image acquisition interface to control imaging parameters and acquisition details, which are subsequently triggered by the automated sequencing at the appropriate time.

In one instance, the microscope is a consumer product and is enclosed along with the fluidic system such that the resulting enclosure acts as a single integrated system with interfaces specified by the CISITS system.

In one instance, the microscope is an OEM format in which the microscopy components are more directly integrated with the automated fluidic system, allowing the entire system to be shipped in an integrated fashion and removing unnecessary mechanical components normally part of a consumer product. An OEM microscope format allows for a more compact enclosure of the automated system. Direct integration facilitates the use of specialized hardware for CISITS automation that directly interface with the imaging components of the microscope. These include, for instance, reagent leakage remediation and detection at the objective or objective turret. They especially include new methods for large area or multiple area acquisitions for CISITS, such as automatic management of the liquid immersion interface between the objective glass and the sample chamber glass. Automatic management of the immersion interface, for instance, water, enables large travel distances of the stage over the objective without loss of immersion and proper refractive index matching, providing the ability for whole section acquisitions, and importantly, allowing the objective head to translate to multiple different wells without loss of the water (or other) immersion interface.

### New software and new methods for orchestrating the automated sequencing process

The previous methods for automated STARmap sequencing described firmware code controlling the individual fluidic components and suggested that this firmware code would be orchestrated by subsequent custom software on a controller computer.

The new CISITS methods expand this process into a multilevel, hierarchical model of abstraction over the system components control software with compatibility for parallel operation; unifies sequencing protocols into a single series of modular commands that accept parameter files specifying the relevant details of the sequencing (from combinatorial to sequential sequencing, and variations of those protocols); provides a user interface for specifying, starting, pausing, and stopping a sequencing run, as well as initiating a wash run; provides a system dashboard accessible over a network that displays sequencing progress, and metrics, including quality checks; and provides methods for a data management system in order to accommodate large datasets that would otherwise block sequencing from proceeding due to space constraints. These new methods provide the means for the rapid, automated, and continuous acquisition of CISITS data.

### Software methods for hierarchical abstraction and system control

The lowest level of firmware code are the individual controllers for each device in the fluidic system, for example the chiller block, pump, or selector valve. An Arduino or microcontroller contains firmware code specifying common operations of the individual hardware components, and provides a series of macros that operate these hardware components according to input parameters. This microcontroller code is interfaced by serial connection with a computer on which image acquisition can occur. CISITS software running on the computer is itself hierarchical, providing multiple abstractions over the control of the various hardware components. In one instance, this software is implemented in the python programming language. A low level module provides an object-oriented model for programmatic interfacing with the hardware microcontroller/Arduino. Initialization of this object ("Robo") opens a serial connection with the microcontroller and provides a handle for subsequent communication through the serial interface, and resets the microcontroller device. Additionally, initialization sets basic parameters for the operation of the devices that are invariant to different sequencing protocols, such as the maximal pump movement speed, maximal volumes, as well as various time constants for equilibration of fluids. The method providing the lowest level of abstraction in this object is a send command method, which communicates a properly formatted serial command string to the microcontroller, triggering the appropriate control macro. Higher level methods of the object orchestrate commands to the microcontroller that execute discrete actions of the fluidic system, including getting and setting the chiller temperature, waiting until the chiller has reached a target temperature, moving pump contents to waste, loading an inputted reagent of a specified volume to a specified sample, emptying a specified sample well of a specified volume to waste, and moving reagent between reagent containers (normally used for cleaning). A final key method of the object is closing the serial connection to the microcontroller. By establishing and holding this serial connection with the Robo object, the software blocks access of other programmatic threads to the microcontroller, ensuring that a single thread can orchestrate the fluidic hardware at a time.

This control object is initialized and its methods are called by a sequencing module which provides a higher level framework for the specification the sequencing protocol. In one instance of the CISITS software methods, the sequencing commands are orchestrated in a "execute-block" paradigm, in which individual operations are executed sequentially and the sequencing procedures wait until the program has completed each command. This provides a convenient and robust method for preventing conflicting commands from acting on the system simultaneously, especially during incubation times, which can simply block execution of the program for an allotted time. In this paradigm, progression through the CISITS protocol, including addition and removal of reagent and incubation times happen strictly sequentially following a script-like program execution. Multiple washes or additions of reagents occur within programmatic for loops, all of which are encapsulated in a programmatic loop across sequencing rounds.

In another instance of the CISITS software methods, the sequencing control for parallel samples is executed as a multithreaded application, in which individual processing threads acquire locks on the sequencing hardware and execute the required sequencing steps asynchronously, one thread per sample well. In this mode of operation, imaging and fluidic movement can occur simultaneously. In another instance, threads operate asynchronously but communicate to optimize timing of sequencing actions and meet constraints of strict incubation times in the chemistry.

In another instance of the CISITS software methods, the sequencing occurs in a state-dependent event-loop paradigm, in which the sequencing module describes a progression through a series of states, some of which may occur in parallel, and an event loop executes this progression according to the state of the system, the program's position in the state flow, and the availability state of the system components. This paradigm is especially suited for interleaved or parallel operation of the sequencer across multiple samples, in which all samples are not absolutely synchronized in their stage of sequencing. For example, a sample incubation command may execute such that a timer is set and checked for a given sample, and when the timer is complete the program may continue with that sample; however, in the meantime, the state of the pump or valves may be available and commanded for operations on other samples while the first sample is incubating.

The sequencing module contains checks for nominal operation of the sequencer, as communicated by the sequencer hardware, such as proper movement of fluids or accidental spillage, and blocks further execution of the sequencing protocol when necessary. The sequencing module also logs its actions, along with a timestamp, to a log file, and provides information in a format that a dashboard can parse the process information into system status and progress. The sequencing loop or event loop also includes checks for interface files that communicate to the sequencing thread that sequencing should be either paused or stopped. In the case of pausing, the sequencing module checks every several seconds for the continued presence of a pause file, and proceeds with sequencing if it is no longer found. In the case of a stop interface file, the sequencing module reports that a stop was found to the log, resets the device, and exits. Generally, when the sequencer begins operation it writes a file denoting that it is running; when it completes or exists operations it removes that file.

Finally, the sequencing module and its commands for executing CISITS is wrapped by a high level graphical user interface. Provided that the sequencer is not already in use, the user interface can execute the sequencing (or washing) module as an independent programmatic thread. Additional threads can be automatically launched by this interface if they are not already running, including a sequencing progress dashboard thread and a data management thread.

### Software methods for specifying parameters of CISITS sequencer actions

The sequencing module of the CISITS control software is parameterized by settings specific for a given sequencing protocol, such as combinatorial or sequential sequencing. The methods are designed such that a specification file for the different sequencing methods fully parameterizes the commands of the sequencer module. Default settings provide sufficient robustness for automated execution of the sequencer, but the user interface provides an advanced mode in which these settings may be modified and saved. This model in which parameters are separated from the main sequencing methods enables recording, reproducibility and reuse of parameters and simplifies the software to a common sequencing module.

### Software methods for graphical user interface control of sequencing

The graphical user interface (GUI) is the main interface between a user and the CISITS sequencer. It displays the sequencer status when running and provides buttons for pausing or stopping the operation of the sequencer. The sequencing module, while running on a separate thread, repeatedly checks for files in its directory that indicate whether sequencing should be paused or altogether stopped, enabling communication between the user interface thread and the sequencing thread, which are otherwise independent once sequencing has started. When sequencing or washing is not in progress, the GUI provides buttons for starting a new sequencing run and for starting a new wash run. It also indicates if a wash should be performed based on the elapsed time since the last CISITS sequencing run was performed. When a wash is initiated (the wash button is pressed), the GUI displays a message to the user to place the wash reagent tray and wash sample container into the sequencer. Once the user has pressed a button confirming that these are in place, a wash cycle proceeds and the user is notified on the GUI of any errors. When sequencing is initiated (the sequencing button is pressed), the user is provided with a text input box for the name of the experiment, a dropdown list to select either combinatorial or sequential sequencing, and a dropdown list for indicating the number of rounds to perform. The user can also click a button to display advanced options including text input boxes showing the default sequencing parameters for combinatorial or sequential sequencing, can load parameters from a saved parameter file, and can input different values and a filename to save a new set of custom sequencing parameters. The user can set the directory or location to which data should be transferred, including the option to set up automatic transfer to cloud storage. Following the entry of relevant information on the GUI, a continue button is pressed to begin sequencing, or a cancel button is pressed to return to the home screen. Following pressing the start button, the GUI briefly opens an interface object to the fluidic system, checks if the chiller is sufficiently cold already, and begins cooling the chiller if it is not already cold, before releasing the connection to the fluidic system. The GUI then directs the user on a subsequent screen through the rest of the sequencing set up steps, including set up of imaging parameters and loading of reagents into the machine. Finally, a start button can be pressed to begin sequencing actions, in which the sequencing module is evoked as a thread with the inputted or selected parameters, and at which point the GUI ensures that a local server thread is running to provide a network-accessible sequencer status dashboard and that a data transfer thread is running. After completion of main sequencing actions and preliminary washing, the GUI communicates a message to the user to replace the sample container with the wash sample container, and clicking a proceed button performs the final wash cycles of the sequencer.

### New software methods for system status dashboard

Initialization of sequencing by CISITS software also launches a web server thread, which serves a dashboard at the sequencer computer that is accessible over the local network. This dashboard displays the status of the sequencer including any errors, progress of the sequencer on a given sample or on several samples, including which round of CISITS is being performed, what the elapsed time is, estimates for time until completion, data on round to round completion time, data on image acquisition time, and quality control metrics depending on whether combinatorial or sequential sequencing is performed, for example, for combinatorial sequencing, the distribution of number of dots detected on the first round per field of view per area, median dot size, distribution of dots across color channels, and round-to-round registration distances; for sequential sequencing, for example, the correlation of signal across channels within a round, or the round-to-round correlation of signal within color channels. To prevent unwanted network access to this dashboard, the dashboard requires a user log in to view, the server limits the number of network requests it can receive from an IP address block to reduce denial-of-service attacks on the acquisition computer, and the dashboard does not provide any control over the sequencer. Data from the dashboard is provided by the sequencer module, which records actions and times via saved files, for example via log files, and external processing pipelines, that computer quality control data on sequencing data as they become available and provide the dashboard with a network location where quality control data can be obtained.

### New software methods for CISITS data acquisition and management

Large area CISITS sequencing runs, large numbers of sequencing rounds, and multi-sample or multi-chamber parallel sequencing all produce data per sequencing round per sample that can fill much of a large hard drive. For instance, a single tiled microscope acquisition of an area of interest of a CISITS sample may range from several hundred Gigabytes to several Terabytes. Thus, it is essential that software methods aggressively move data off of an acquisition hard drive and onto either a very large local storage drive pool, or onto a large buffer drive and subsequently onto a large networked storage drive or cloud storage for subsequent downstream processing. These software methods can run as an independent thread that check for available data and move them off of the acquisition drive and to their storage destination. Failure to properly manage data can result in a rapid loss of space on an acquisition drive and the stoppage of sequencing, thus, these methods are essential for large scale and high throughput sequencing.

### New software methods for integration with microscopy platforms

CISITS software methods integrate the fluidic system operation with the operation of a microscopy platform. In some instances, the microscopy platform provides an independent image acquisition GUI, which can be used to set up the desired imaging parameters, fields of view, tiles, and locations, and triggered or initiated by serial, TTL, REST, or other API commands from the sequencing software module. In this case the sequencing module automatically communicates with the image acquisition software to initiate a user specified imaging protocol at the correct time in the sequencing chemistry protocol, and waits for acquisition of that sample to finish before starting any subsequent image acquisitions or procedures. Alternatively, as in the case of parallel sample well operation, the software may begin an image acquisition and meanwhile proceed with other fluidic operations on other samples, while periodically querying the image acquisition software for the completion status of the ongoing acquisition, before starting a new queued acquisition. In other instances, especially those in which the microscopy platform is more directly integrated with the fluidic system, the CISITS software itself integrates within the GUI sufficient microscope functions for the user to set up the desired image acquisition parameters before sequencing begins. In this case, microscope functions are directly implemented by the CISITS software by wrapping external microscope control and interface libraries, for instance micromanager, or by direct integration with the microscope system's acquisition software. Minimal CISITS software integration with microscopy platforms include setting an acquisition filename prefix, executing on the microscope system a saved imaging protocol, getting the completion status of an acquisition, and for parallel operation of the sequencer across multiple sample wells, getting and setting the stage position. CISITS software controlling sequencing across multiple sample areas allows the user to specify starting positions for each distinct area to be imaged and to tie those areas either to parallel well sequencing or to multiple locations to be imaged sequentially within a single chamber. This is accomplished during imaging set up, in which the software queries the microscope system for the stage position and saves the stage position as a list of acquisition start positions for a given sample, or a list of lists of acquisition start positions, where the outer list are different, parallel-sequenced sample chambers.

### Applications:

*In situ* nucleic acid sequencing techniques from laboratory scale towards clinical and industrial scales are described herein. The sample types these techniques may be applied to include: cultured layers of cells, cultured organoids, or sections of cultured organoids, and animal biopsy sample or postmortem animal tissue. It can be used to record the spatial location of target genes in these samples; the genetic "identity" or "type" of a cell based on a pattern of expressed genes or the presence of marker genes; the location of exogenous genes such as anatomical or functional markers within cells or tissues; changes in RNA expression across experimental conditions; and can identify spatial expression of genes in cells that express other orthogonal markers, including being used in genetic or engineering screens.

### Advantages & improvements over existing methods, devices or materials:

CISITS enables improved robustness and high throughput intact tissue sequencing, combining chemistry improvements to SNR and process robustness, and automation (hardware and software) improvements to throughput and robustness, over the previously described STARmap method.

## Claims

1. A method for *in situ* sequence identification of a nucleic acid in a cell in a tissue sample, comprising:
(a) embedding said tissue sample in a hydrogel;
(b) contacting said tissue sample with a plurality of oligonucleotides under conditions sufficient to allow for specific hybridization;
(c) incubating said tissue sample with a polymerase in an absence of nucleotides for a sufficient time to allow uniform diffusion of said polymerase;
(d) performing an amplification reaction by contacting said tissue sample with nucleotides to generate one or more amplicons from said plurality of oligonucleotides;
(e) linking ribonucleic acid (RNA) of the tissue sample to the hydrogel;
(f) crosslinking said tissue sample, thereby attaching RNA of the tissue sample to the hydrogel; and
(g) imaging said one or more amplicons to identify said nucleic acid.

2. The method of claim 1, wherein
(a) the plurality of oligonucleotides comprises a first oligonucleotide, wherein said first oligonucleotide comprises a first region that binds to said nucleic acid; or
(b) the plurality of oligonucleotides comprises a first oligonucleotide and a second oligonucleotide, wherein said first oligonucleotide binds to said second oligonucleotide, or said second oligonucleotide binds to said nucleic acid, or a combination thereof.

3. The method of claim 1, wherein:
(a) said plurality of oligonucleotides comprises a first oligonucleotide and a second oligonucleotide;
(b) said second oligonucleotide comprises a second region that binds to a second portion of said nucleic acid;
(c) said first region is adjacent to said second region when said first oligonucleotide and said second oligonucleotide are bound to said first portion and said second portion of nucleic acid, respectively;
(d) said first oligonucleotide comprises a third region and said second oligonucleotide comprises a fourth region, wherein said third region is complementary to said fourth region; and
(e) wherein said first oligonucleotide comprises a fifth region and said second oligonucleotide comprises a sixth region, wherein said fifth region is complementary to said sixth region.

4. The method of claim 3, further comprising, subsequent to (d), adding a ligase to said tissue sample to generate a closed nucleic acid circle using said second oligonucleotide.

5. The method of any one of claims 1-4, further comprising, subsequent to (e), clearing said tissue sample of a plurality of cellular components.

6. The method of any one of claims 1-5, wherein said amplification reaction comprises a rolling circle amplification reaction.

7. The method of any one of claims 1-6, wherein said one or more amplicons comprise a barcode.

8. The method of any one of claims 1-7, wherein said tissue sample is a formalin-fixed paraffin embedded tissue sample.

9. The method of any one of claims 1-8, wherein said nucleic acid is ribonucleic acid and said ribonucleic acid comprises messenger ribonucleic acid.

10. The method of any one of claims 1-9, wherein (g) comprises imaging the one or more amplicons using an imaging system.

11. The method of claim 10, wherein said imaging system comprises a light sheet microscope.

12. The method of claim 10, wherein said imaging system comprises a confocal microscope.

13. The method of any one of claims 1-12, wherein said tissue sample comprises a thickness of 50 micrometers (µm) to 200 µm.

14. The method of any one of claims 1-13, further comprising, prior to (c), adding a permeabilization agent to said tissue sample.

## Patentansprüche

1. Verfahren zur In-situ-Sequenzidentifikation einer Nukleinsäure in einer Zelle in einer Gewebeprobe, umfassend:
(a) Einbetten der Gewebeprobe in ein Hydrogel;
(b) Inkontaktbringen der Gewebeprobe mit einer Vielzahl von Oligonukleotiden unter Bedingungen, die ausreichen, um spezifische Hybridisierung zu ermöglichen;
(c) Inkubieren der Gewebeprobe mit einer Polymerase in Abwesenheit von Nukleotiden für eine ausreichende Zeit, um gleichmäßige Diffusion der Polymerase zu ermöglichen;
(d) Durchführen einer Amplifikationsreaktion durch Inkontaktbringen der Gewebeprobe mit Nukleotiden, um ein oder mehrere Amplicons aus der Vielzahl von Oligonukleotiden zu erzeugen;
(e) Verknüpfen von Ribonukleinsäure (RNA) der Gewebeprobe mit dem Hydrogel;
(f) Vernetzen der Gewebeprobe, wodurch RNA der Gewebeprobe an das Hydrogel gebunden wird; und
(g) Abbilden des einen oder der mehreren Amplicons, um die Nukleinsäure zu identifizieren.

2. Verfahren nach Anspruch 1, wobei
(a) die Vielzahl von Oligonukleotiden ein erstes Oligonukleotid umfasst, wobei das erste Oligonukleotid eine erste Region umfasst, die an die Nukleinsäure bindet; oder
(b) die Vielzahl von Oligonukleotiden ein erstes Oligonukleotid und ein zweites Oligonukleotid umfasst, wobei das erste Oligonukleotid an das zweite Oligonukleotid bindet oder das zweite Oligonukleotid an die Nukleinsäure bindet oder eine Kombination davon.

3. Verfahren nach Anspruch 1, wobei:
(a) die Vielzahl von Oligonukleotiden ein erstes Oligonukleotid und ein zweites Oligonukleotid umfasst;
(b) das zweite Oligonukleotid eine zweite Region umfasst, die an einen zweiten Teil der Nukleinsäure bindet;
(c) die erste Region zu der zweiten Region benachbart ist, wenn das erste Oligonukleotid und das zweite Oligonukleotid an den ersten Teil bzw. den zweiten Teil der Nukleinsäure gebunden sind;
(d) das erste Oligonukleotid eine dritte Region umfasst und das zweite Oligonukleotid eine vierte Region umfasst, wobei die dritte Region zu der vierten Region komplementär ist; und
(e) wobei das erste Oligonukleotid eine fünfte Region umfasst und das zweite Oligonukleotid eine sechste Region umfasst, wobei die fünfte Region zu der sechsten Region komplementär ist.

4. Verfahren nach Anspruch 3, ferner umfassend, anschließend an (d), Hinzufügen einer Ligase zu der Gewebeprobe, um einen geschlossenen Nukleinsäurering unter Verwendung des zweiten Oligonukleotids zu erzeugen.

5. Verfahren nach einem der Ansprüche 1-4, ferner umfassend, anschließend an (e), Befreien der Gewebeprobe von einer Vielzahl von zellulären Komponenten.

6. Verfahren nach einem der Ansprüche 1-5, wobei die Amplifikationsreaktion eine Rolling-Circle-Amplifikationsreaktion umfasst.

7. Verfahren nach einem der Ansprüche 1-6, wobei das eine oder die mehreren Amplicons einen Barcode umfassen.

8. Verfahren nach einem der Ansprüche 1-7, wobei die Gewebeprobe eine Formalin-fixierte Paraffin-eingebettete Gewebeprobe ist.

9. Verfahren nach einem der Ansprüche 1-8, wobei die Nukleinsäure Ribonukleinsäure ist und die Ribonukleinsäure Messenger-Ribonukleinsäure umfasst.

10. Verfahren nach einem der Ansprüche 1-9, wobei (g) Abbilden des einen oder der mehreren Amplicons unter Verwendung eines Bildgebungssystems umfasst.

11. Verfahren nach Anspruch 10, wobei das Bildgebungssystem ein Lichtscheibenmikroskop umfasst.

12. Verfahren nach Anspruch 10, wobei das Bildgebungssystem ein konfokales Mikroskop umfasst.

13. Verfahren nach einem der Ansprüche 1-12, wobei die Gewebeprobe eine Dicke von 50 Mikrometer (µm) bis 200 µm umfasst.

14. Verfahren nach einem der Ansprüche 1-13, ferner umfassend vor (c) Hinzufügen eines Permeabilisierungsmittels zu der Gewebeprobe.

## Revendications

1. Procédé d'identification de séquence *in situ* d'un acide nucléique dans une cellule dans un échantillon de tissu, comprenant :
(a) l'incorporation dudit échantillon de tissu dans un hydrogel ;
(b) la mise en contact dudit échantillon de tissu avec une pluralité d'oligonucléotides dans des conditions suffisantes pour permettre une hybridation spécifique ;
(c) l'incubation dudit échantillon de tissu avec une polymérase en l'absence de nucléotides pendant un temps suffisant pour permettre une diffusion uniforme de ladite polymérase ;
(d) la réalisation d'une réaction d'amplification en mettant en contact ledit échantillon de tissu avec des nucléotides pour générer un ou plusieurs amplicons à partir de ladite pluralité d'oligonucléotides ;
(e) la liaison de l'acide ribonucléique (ARN) de l'échantillon de tissu à l'hydrogel ;
(f) la réticulation dudit échantillon de tissu, fixant ainsi l'ARN de l'échantillon de tissu à l'hydrogel ; et
(g) l'imagerie dudit ou desdits amplicons pour identifier ledit acide nucléique.

2. Procédé selon la revendication 1, dans lequel
(a) la pluralité d'oligonucléotides comprend un premier oligonucléotide, ledit premier oligonucléotide comprenant une première région qui se lie audit acide nucléique ; ou
(b) la pluralité d'oligonucléotides comprend un premier oligonucléotide et un second oligonucléotide, ledit premier oligonucléotide se liant audit second oligonucléotide, ou ledit second oligonucléotide se liant audit acide nucléique, ou une combinaison correspondante.

3. Procédé selon la revendication 1, dans lequel :
(a) ladite pluralité d'oligonucléotides comprend un premier et un second oligonucléotide ;
(b) ledit deuxième oligonucléotide comprend une deuxième région qui se lie à une deuxième partie dudit acide nucléique ;
(c) ladite première région est adjacente à ladite deuxième région lorsque ledit premier oligonucléotide et ledit second oligonucléotide sont liés respectivement à ladite première partie et à ladite seconde partie d'acide nucléique ;
(d) ledit premier oligonucléotide comprend une troisième région et ledit second oligonucléotide comprend une quatrième région, ladite troisième région étant complémentaire de ladite quatrième région ; et
(e) dans lequel ledit premier oligonucléotide comprend une cinquième région et ledit second oligonucléotide comprend une sixième région, ladite cinquième région étant complémentaire de ladite sixième région.

4. Procédé selon la revendication 3, comprenant en outre, après (d), l'ajout d'une ligase audit échantillon de tissu pour générer un cercle d'acide nucléique fermé en utilisant ledit second oligonucléotide.

5. Procédé selon l'une quelconque des revendications 1 à 4, comprenant en outre, après (e), l'élimination, dudit échantillon de tissu, d'une pluralité de composants cellulaires.

6. Procédé selon l'une quelconque des revendications 1 à 5, dans lequel ladite réaction d'amplification comprend une réaction d'amplification en cercle roulant.

7. Procédé selon l'une quelconque des revendications 1 à 6, dans lequel ledit ou lesdits amplicons comprennent un code à barres.

8. Procédé selon l'une quelconque des revendications 1 à 7, dans lequel ledit échantillon de tissu est un échantillon de tissu incorporé dans de la paraffine, fixé au formol.

9. Procédé selon l'une quelconque des revendications 1 à 8, dans lequel ledit acide nucléique est un acide ribonucléique et ledit acide ribonucléique comprend un acide ribonucléique messager.

10. Procédé selon l'une quelconque des revendications 1 à 9, dans lequel (g) comprend l'imagerie du ou des amplicons en utilisant un système d'imagerie.

11. Procédé selon la revendication 10, dans lequel ledit système d'imagerie comprend un microscope à feuille de lumière.

12. Procédé selon la revendication 10, dans lequel ledit système d'imagerie comprend un microscope confocal.

13. Procédé selon l'une quelconque des revendications 1 à 12, dans lequel ledit échantillon de tissu comprend une épaisseur de 50 micromètres (µm) à 200 µm.

14. Procédé selon l'une quelconque des revendications 1 à 13, comprenant en outre, avant (c), l'ajout d'un agent de perméabilisation audit échantillon de tissu.
